# EUROPEAN PATENT SPECIFICATION

(11) **EP 0 861 651 B1**
(45) Date of publication and mention of the grant of the patent: **17.04.2002**
(21) Application number: 98108100.3
(22) Date of filing: 17.12.1992
(51) Int. Cl.: A61H 23/04

(54) **Pneumatic compression device and methods for use in the medical field**
Pneumatische Kompressionseinrichtung sowie Verfahren zur Anwendung auf medizinischem Gebiet
Dispositif de compression pneumatique et méthodes destinées à être utilisées dans le domaine médical

(30) Priority: 17.12.1991 US 809423
(43) Date of publication of application: 02.09.1998
(62) Divisional of application: 93902661.3
(73) Proprietor: KINETIC CONCEPTS, INC., San Antonio, TX 78265-9508 (US)
(72) Inventor: Lina, Cesar Z., University City, Texas 78148 (US)
(74) Representative: Moreland, David, Dr.

(56) References cited:
- EP-A- 0 221 636
- GB-A- 2 103 842
- US-A- 4 133 311

## Description

### BACKGROUND OF THE INVENTION

### Field of the Invention:

There are a few basic aspects of the present invention, namely a method and apparatus for cyclic compression of a body's extremities, together a related system with a housing for containing and mounting the device on the foot board of a hospital bed.

### Related Art:

Medical devices that apply cyclic pressure to a person's legs, arms, hands and/or feet are very old and well-known in the art. Many have employed pulsating pads or plungers for improving circulation. Others have used hydraulic and pneumatic bladders for similar purposes. The shapes, sizes, and composition of such bladders and pads are widely varied, depending largely on their particular application.

Man has known the fundamental principle of most cyclic compression devices for thousands of years. They are merely a more recent embodiment of the old art of massage, which has been used to stimulate circulation since prehistory. Use of mechanical devices to effect the massaging action is obviously more recent, but has a clear history of more than 150 years.

Full understanding of the mechanism involved in this form of improving blood flow is more recent but has not fundamentally changed the devices used to accomplish this result. Veins are now known to contain a series of one-way check valves along their length. Thus, when pressure is applied, compressing a vein, the fluid expelled therefrom can only proceed in the direction of normal circulation. When such compression is relaxed, the vein returns to its normal circular cross-section, and the flow of blood into the vein is increased until it reaches its normal state of back pressure. Repeating this cycle in a cyclic fashion thus increases blood flow in the normal direction of circulation.

Such compression/decompression cycles occur naturally in humans as part of the action of the muscles and flexure of the limbs. It has been known for many years that the arch of the foot includes a large venous plexus (or group of veins). It is also known that this venous plexus is compressed during normal walking or running, thereby stimulating circulation. This efficient circulation aid is a marvelous design by our Creator, as its effect is greatest when the leg muscles (the largest muscles in the body) are in action and need the oxygen supplied by enhanced circulation.

For these and other reasons, the foot has long been known as an effective site for applying cyclic pressure. For instance, many devices such as Massator's "PediPulsor" improve circulation by positioning a pulsating, dome-shaped pad in the arch of the foot. Many others have targeted the arch of the foot with flexible pneumatic chambers. A partial sampling of such pneumatic devices that target the arch of the foot includes Japanese Utility Model No. 72-10392, U.S. Patent No. 4,614,180 in the name of Gardner and Fox, and U.S. Patent No. 4,941,458 in the name of Taheri (although the Gardner and Fox patent focuses much of its disclosure on a different type of venous pump ― a pump achieved by periodically flattening the arch rather than simply compressing it).

Many others have long recognized that the foot contains veins that can be massaged or pumped to provide better circulation. Some examples are: L.E. Corcoran, who states in his U.S. Patent of April 7, 1959 that massaging the soles of the feet "promotes a beneficial degree of circulation"; Richard Dillon M.D. whose Journal of Vascular Diseases, January 1986 report on treatment of circulation-impaired patients states "compression boot therapy enjoys a 173 year history;" and P. Gaskell M.D. and J.C.W. Parrot M.D. whose Surgery, Gynecology, and Obstetrics, April 1978 report shows a high level of understanding of the process of venous pumping with pulsed air by stating "We have found that the boot covering the foot alone is simpler, less cumbersome, and gives a greater reduction of venous pressure than either a large cuff which covers the whole calf or a boot which includes the calf and the foot".

Such devices and many other medical devices are typically auxiliary devices which may or may not be employed on each patient. Because of its auxiliary nature, it is beneficial for such devices to be compact, portable and easily employed and stored adjacent a hospital bed. Other devices have addressed such concerns with hooks that allow the device to be hung on the foot board of a bed. However, because of the wide range of foot board sizes that must be accommodated, such hooks either have to be customized for certain foot boards, or else the snugness and security of the fit must be compromised.

EP 0 221 636 (Electro-Biology, Inc) discloses a non-invasive technique and apparatus for artificially stimulating the venous-return flow of blood from the foot by inducing fast-rising pulsed squeezing or necking-down of the vessels of the venous-pump mechanism within the foot. The stimulation results from transient flattening of the plantar arch, in that an induced transient spread of the heel with respect to the ball of the foot stretches, and therefore necks-down involved blood vessels; stimulation also results from such a squeeze of the plantar-arch region as to concurrently squeeze the involved blood vessels. Cyclically inflatable devices, local to the foot-pump region, are disclosed for inducing either or both of the indicated actions.

US 4,133,311 (Karczewski) discloses a reusable ankle support structure comprising an inner layer of a resilient material, such as neoprene rubber, and an outer layer of a relatively non-resilient but flexible material, such as denim cloth, that is secured as a pair of mirror image sections to the outside surface of the inner layer. Each of the outer layer sections includes an integral, elongated flexible strap extending therefrom. The resilient inner layer is snugly wrapped around the ankle of a human with the straps to the front of the ankle. The straps are snugly wrapped around the foot crossing over the top of the foot and again under the arch of the foot, pulled upwardly on opposite sides of the ankle and then secured under tension to the sides of the structure. The straps thus combine with the inner layer to provide stirrup-like support against eversion and in inversion of the ankle. Hook and loop fastening panels may be secured to facilitate its application.

Despite the long history of technological developments of such housings and compression devices, man continues in his manifest pursuit of the ideal system which balances maximum therapeutic benefit with practicality and patient comfort.

Many other problems, obstacles and deficiencies faced in these fields and/or addressed by the present invention will be apparent to those skilled in that art, especially in light of the further descriptions herein.

It is a primary object of the present invention to provide a new and improved cyclic compression system which overcomes the problems and deficiencies of the prior art.

It is also an object of the present invention to provide a novel housing which can be conveniently handled and simply but securely mounted to a hospital bed or the like. Another object of the invention is to produce a simple, compact, inexpensive pump and housing which are capable of yielding substantial therapeutic and prophylactic benefits despite their size and simplicity.

The present invention is directed toward accomplishing such objects and others and improving upon the teachings of the prior art by uniquely combining, refining and modifying various concepts and features to provide a significant advancement in the field. A primary object of the invention is to provide a small, lightweight and comfortable but durable device which helps prevent and/or solve many of the problems associated with impaired or poor circulation.

Another object includes providing a pneumatic device which encloses only limited portions of the foot, especially those portions which may be readily compressed to improve circulation. Related objects include providing comfort and moisture control and avoiding the need for accessories such as additional stockings, wraps, sandals, straps, and the like, which have been required by the prior art.

Another object of the present invention is to provide an intermittent compression device requiring a minimum volume of air per pulsation.

Another object is to provide a blood circulation aid which will fit a wide variety of patients without requiring any modification or adjustments.

Another object is to provide a device of great simplicity and ease-of-use in contrast to other devices designed for the purpose of aiding blood flow in the feet and legs.

Another object of the invention is to provide a blood flow improvement device which, due to its inherent low manufacturing cost, is practical to use as a disposable item rather than cleaning and reusing.

Still another object is to provide a cyclic compression device which achieves maximum compression in a manner that minimizes anxiety and discomfort to the patient.

Other objects relate to the convenience of the product. It is desired to provide a complete system that can be hand carried to the patient and easily and securely deployed without needing to be concerned about compaticility with the bed on which the product is to be deployed. A compact, easily-stored and minimally-obtrusive product is also sought.

The present invention addresses the foregoing and many other objects by providing a medical apparatus as claimed in claim 1.

The invention provides an ingenious apparatus that integrates a compression bladder and its entire mounting, stabilizing and adjustment systems into a simple and economical construction coupled with a control system and housing that greatly surpass prior devices both in utility and ingenuity.

The present invention will typically comprise a foot wrap device made from two sheets of fabric sewn or welded together to form an inflatable pocket or bladder in part of a main body area. One aspect of the invention relates to its roughly T-shaped configuration, with at least one extension from the main body area for encompassing the foot's arch. A second extension preferably extends from the main body in a direction opposite the first. A third extension from the main body is roughly perpendicular to the arch-encompassing extensions, for embracing the back of the heel. In the preferred embodiment, both inner and outer fabric layers are cut from the same pattern.

Fasteners formed integral with two of the extensions enable releasable application on the foot. Preferably, such fasteners include Velcro hook connectors, and the outer surface of the foot wrap is formed of Velcro loop material (or the equivalent) for mating with the hooked fasteners. The inner layer of the foot wrap is a vapor permeable material having greater elasticity than the outer layer. Both fabrics are preferably impermeable to air and capable of being fused together by heat welding. A filling tube is sealed into said inflatable bladder through the outer fabric layer.

In the preferred embodiment, the complete foot wrap weighs only a few ounces and is soft and pliable. When the device is properly applied, the inflatable bladder lies under the arch of the foot. One extension wraps over the instep to completely surround the foot and fastens to the outside surface of the main body section. The second extension wraps around behind the heel and also fastens to the outside surface of the main body section, thus securely holding the device in place on the foot to hold the bladder in place when it is inflated. Fluid for such extension is supplied in a pulsed sequence selected for frequency and intensity by the physician from one of the pump/control systems well known in the art.

Certain embodiments uniquely integrate above aspects of the invention together with a cushioned strap for minimizing localized pressure concentrations related to said surface, thereby inhibiting skin breakdown on vulnerable areas of the foot.

The present invention also includes a unique pressure source for supplying pressure to the referenced foot wraps. Also provided is pressure feedback control of the pressure supplied by the pressure source, even during the then-current compression cycle. The feedback system overcomes the diffiulties inherent in its objects by incorpating various spike eliminators, as well as error detection means for alarming the operator of error conditions such as kinked hoses or disconnected foot wraps. The spike eliminator may include an accumulator, possibly in combination with a flow restrictor, for preventing pressure sensors from measuring the initial surge of pressure that is common with rapid inflation compression devices.

More particularly, the pressure sensing and control system may include an analog to digital converter and a digital micro-controller that controls a plurality of solenoid valves to adjust the supplied pressure. The micro-controller is interfaced through an operator console having liquid crystal display and programmable keys to enter user settings, which are then stored using various memory devices including electronically erasable programmable read only memory.

The combination of all the foregoing in an operative system is thought to be very beneficial, especially packaged in a housing that can be releasably mounted on the foot board of a standard hospital bed by means of a pair of opposite, retractable mounting hooks having sloped surfaces for biasing the source toward said foot board and having knobs at the distal ends thereof for helping retain the hooks on the foot board.

Numerous other features, advantages, and objects of the invention are set forth and will be evident from the following more detailed descriptions of certain preferred embodiments, particularly when considered in light of the prior art together with the accompanying drawings and appended claims.

### BRIEF DESCRIPTION OF THE DRAWINGS

Presently preferred embodiments constructed according to the teachings of the present invention are hereinafter described in more detail. To those of ordinary skill in the art, the invention will become more readily understood from the specifications of those embodiments, particularly when considered in light of the appended claims and with further reference to the accompanying drawings, wherein like numerals refer to like elements throughout, and wherein:

Figure 1 shows a perspective view of the basic elements of the presently preferred embodiment.

Figure 2 shows the outer surface of one of the foot wraps 1A pictured in Fig. 1 as it is laid out flat.

Figure 3 shows a view of the inner surface of the foot wrap 1A laid flat.

Figure 4 shows a top view of the foot wrap 1A in place on a human foot.

Figure 5 shows a side view of the foot wrap 1A in place on a human foot.

Figure 6 shows a cross section of the foot wrap 1A sectioned along plane "A-A" shown in Figure 2.

Figure 7 shows the same cross-section as in Figure 6 except that bladder 9 is shown inflated in Fig. 7.

Figures 8A - 8D illustrate the manner of operatively applying a foot wrap 1A' (similar to foot wrap 1A of Figs. 1-7) to a foot 100.

Figure 9 shows a schematic layout of the electrical and pneumatic systems of the preferred embodiment shown in Fig. 1.

Figures 10 - 12 show flow charts which characterize the operation of the preferred embodiment shown in Fig. 1.

Figure 13 shows a top view of the preferred embodiment's pump housing 20, which also functions as a carrying case and as a means for mounting the pump on the foot board of a hospital bed or the like.

Figure 14 shows a front elevation view of the pump housing 20, without certain details.

Figure 15 shows a rear elevation view of the pump housing 20.

Figure 16 shows a bottom view of the pump housing 20.

Figure 17 shows a left side elevation view of the pump housing 20, with retractable mounting hooks 21 in their fully retracted position as in Figs. 13-16.

Figure 18 shows the left side elevation view of Fig. 17, except that the retractable mounting hooks 21 are in their fully extended position in Fig. 18, as also pictured in Fig. 1.

Figure 19 shows an exploded view of the rear portion of pump 20.

Figure 20 shows an exploded view of the front portion of pump 20, illustrating many of the particular embodiments and relative configurations of the components schematically shown in Fig. 9.

Figures 21-23 show alternative embodiments to the foot wrap 1A shown in Fig. 3.

### DETAILED DESCRIPTION OF PREFERRED EMBODIMENTS

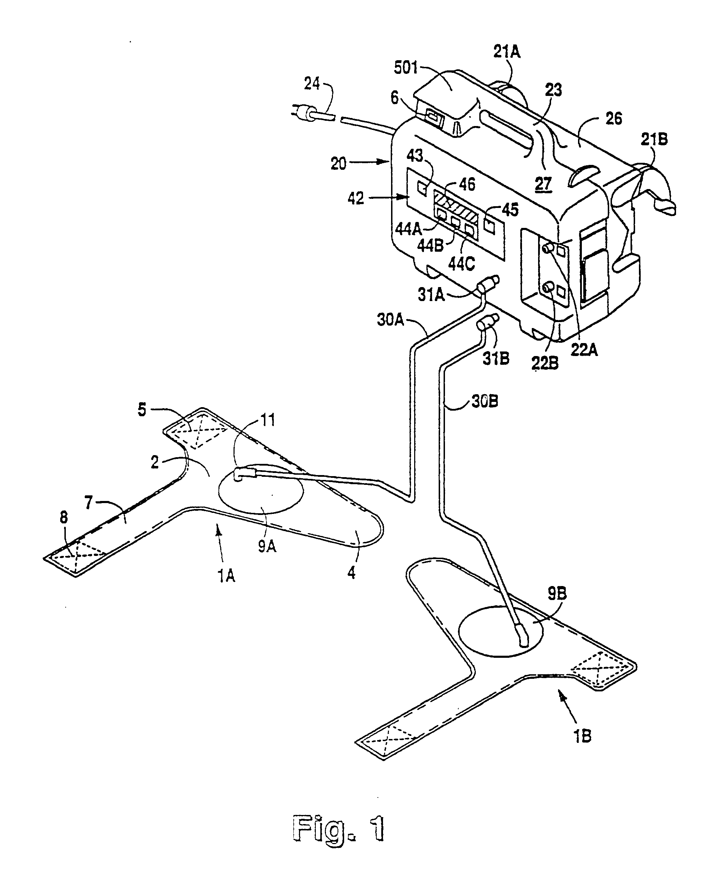

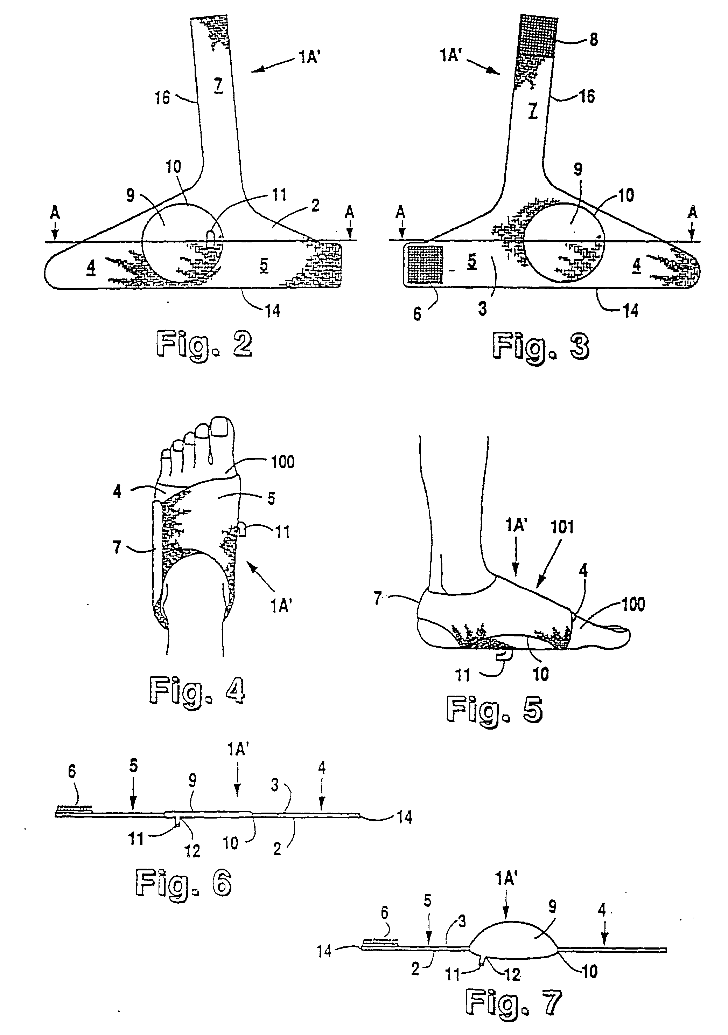

Referring to Figure 1 of the drawings, there is shown an isometric perspective view of the basic elements of the presently preferred embodiment. That embodiment, referred to as cyclic compression system 500, basically comprises a pump unit 501, two foot wraps 1A & 1B, power cord 24, and connecting hoses 30A and 30B. In many respects that embodiment is intended to be substantially identical to a product known in the marketplace as the "PlexiPulse" unit manufactured by Applicant Kinetic Concepts, Inc., San Antonio, Texas. The "PlexiPulse" unit is now available to customers of NuTech, which is affiliated with Applicant and also has offices in San Antonio, Texas. Although the following description is intended to be complete within itself and with reference to its corresponding Co-Pending U.S. Application (referred to previously), further reference to said PlexiPulse unit may further enable those of ordinary skill in the art to make and use the inventions hereof.

The pump unit 501, which is AC-powered via a conventional power cord 24, operates to cyclically inflate and deflate bladders 9A and 9B formed in foot wraps 1A and 1B, to intermittently compress the foot. The unit is described most particularly for application on the foot, as many aspects of the invention can be appreciated there. Nonetheless, regardless whether it is mounted on the calf, the foot, hand, or the site of any other venous plexus, such intermittent compression may be beneficial for a wide variety of indications, including among others: venous stasis, poor circulation, postoperative pain and swelling, edema, cutaneous ulceration, and elevated compartmental pressures. It may also serve to prevent deep vein thrombosis and reduce wound healing times. More particular aspects of preferred operation of the system 500 will be described further herein.

For purposes of this description, reference may be made to "upper", "lower", "forward", "backward", "left", "right" and "horizontal" features when referring to certain drawings. Such references and others like them are made principally to render the description more easily understood in view of the upper and lower orientations in the drawings and in view of a reference orientation of system 500 or its subject. As will be evident to those of ordinary skill in the art, such orientations are not necessarily maintained throughout the operation of the described embodiments, much less would they be required for utilization of the invention.

Referring to Figures 2-7, a foot wrap 1A' is shown, first laid flat and then in relation to a foot 100 during normal operation. Foot wrap 1A' is very similar to foot wrap 1A (shown in Figs. 1, 8 and 9) and thus has parts which are numbered the same as in the other figures. One notable difference is that the tab 7 of wrap 1A would be more precisely perpendicular to the line "A-A" than the slightly sloping direction of tab 7 of wrap 1A'. The angle of the spicket (or nozzle) 11 relative to the foot wrap 1A or 1B (or 1A' or 1B') may also vary from one foot wrap to the next. The spicket angle pictured in Figure 1 is considered most advantageous, tending to bias hose 30A in a direction for avoiding kinking of that hose 30A. Aside from the exceptions noted above, the descriptions of foot wrap 1A' are equally applicable to foot wrap 1A, and similar (but opposite) descriptions apply equally to foot wraps 1B and 1B' which are merely mirror images of their counterparts 1A and 1A'.

The foot wrap 1A' is ideally shaped for application on the left foot, whereas foot wrap 1B (shown in Figure 1) is ideally shaped for application on the right foot. The simplicity of the shape does allow for either wrap 1A or 1B to be interchangeable between left and right feet, if necessary, and it can fit on other body portions as well.

In Figure 2, foot wrap 1A' is shown open (i.e., laid out flat), with the outer surface of foot wrap 1A' facing the viewer. Figure 3 is a view from the opposite side showing the inner surface of foot wrap 1A'. Figure 6 is a cross section along line "A-A" of Figure 3 and generally illustrates the construction of the foot wrap. The foot wrap 1A' is generally formed of two sheets 2 and 3 which are bonded together to form a bladder 9 with tabs 4, 5 and 7 extending away from the bladder 9. Foot wrap 1A' also includes a fluid inlet 11 (also referred to as "fitting 11") for inflating and deflating the bladder 9, as well as fasteners 6 and 8 for releasably securing the wrap 1A' on a foot (designated as foot 100 in the drawings). These basic components and others herein are readily available through numerous manufacturers.

Referring primarily to Figure 6, sheet 2 is preferably cut from a robust, non-stretch fabric. The outer surface of sheet 2 (i.e., the surface facing away from sheet 3) has loops like those found on Velcro loop material, which are compatible to releasably engage Velcro hook material. The interior surface of sheet 2 (i.e., the surface facing toward sheet 3) is heat-weldable. Sheet 2, thus, is referred to as a sheet of laminated loop fabric that forms the outer sheet of wrap 1A'. In the presently preferred embodiment, sheet 2 is a continuous-loop nylon fabric manufactured by Guilford, USA and available under the trade designation "Tricot". Sheet 2 is also laminated with polyurethane in a conventional manner, to seal it and render it RF-weldable.

Preferably, although each of the sheets 2 and 3 are air impermeable, they are each also formed of vapor permeable fabric. Other commercially-available fabrics, such as that marketed as low air loss "GoreTex" by the W. L. Gore Company, have long been known to provide similar benefits in other contexts. Their vapor-permeability serves to enable moisture from foot 100 to evaporate despite the foot wrap 1A'. This is especially preferable for sheet 3 so that perspiration adjacent bladder 9 can be evacuated from the site by the fluid that inflates and deflates bladder 9. The removal of surface moisture forming on the patient's skin beneath the foot wrap is beneficial since it helps promote the maintenance and healing of skin conditions, especially during protracted use. As will be evident to those skilled in the art, though some aspects of this invention can still be appreciated with materials of limited porosity, generally, so long as foot wraps 1A and 1B remain inflatable.

Sheet 3 is preferably cut from the same or a similar pattern as sheet 2, so that it matches neatly with sheet 2. The manufacturing process may be simplified by first joining the sheets 2 and 3 together (as described elsewhere herein) and then cutting the border of each sheet. The cutting process may also be simplified by welding the two sheets together while simultaneously heat-cutting the border of the fabric with the same die (as is common in the art), although this process is not always successful due to the compositions of the sheets.

Sheet 3 is preferably also an elastic fabric, so that it expands more than outer sheet 2 when bladder 9 is inflated (as shown in Figure 7). The inner surface of sheet 3 (i.e., the surface facing toward sheet 2) is laminated with polyurethane, thereby sealing it and rendering it heat-weldable to enable bonding with the inner surface of sheet 2. It is important that the outer surface of sheet 3 (i.e., the surface facing away from sheet 2) is soft and comfortable against the skin, as that surface is likely to be in contact with the patient's skin during use. In the preferred embodiment, sheet 3 is a laminated lycra material that meets the foregoing characteristics.

As will be evident from this description to those of ordinary skill in the art, other fabrics may be substituted for the preferred fabrics of sheets 2 and 3 with related sacrifices of various aspects of this invention. Less costly, substantially inelastic nylon fabrics could be used for both sheets 2 and 3 while still appreciating therapeutic use of the foot wraps 1A and 1B.

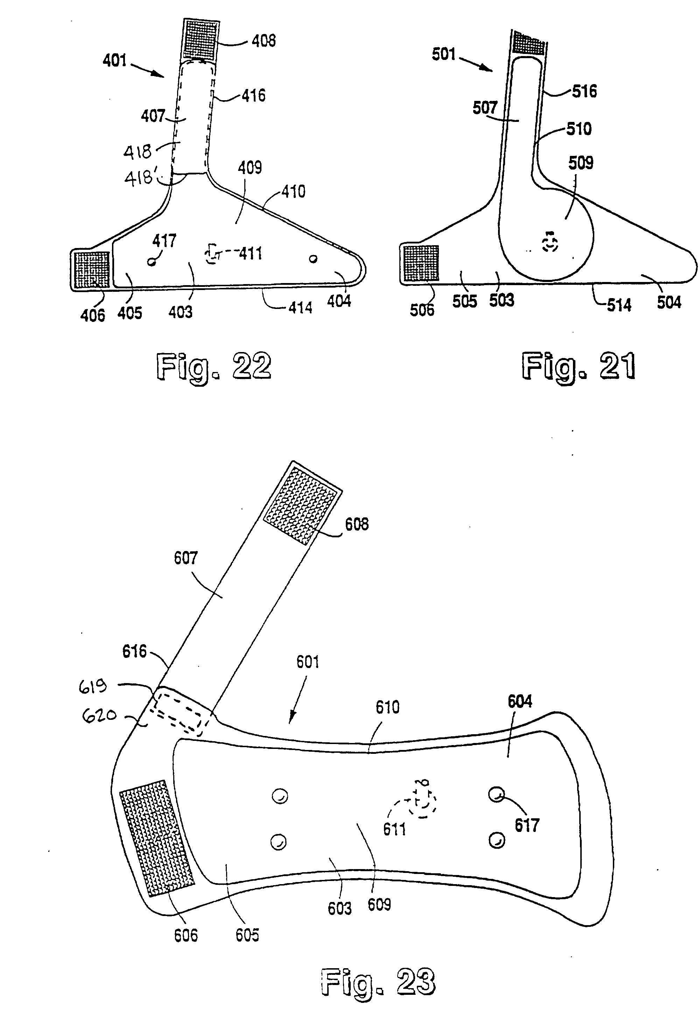

Bladder 9 is formed between sheet 2 and sheet 3 by weld line 10. Weld line 10 is a closed line so that it completely surrounds and thereby defines a closed area on each of sheets 2 and 3. Thus, bladder 9 is a sealed bladder, the only inlet or outlet of which is provided by a tubular connector fitting 11 (described below). Bladder 9 is provided in foot wrap 1A' to apply pressure on the foot 100 when the wrap 1A' is secured on the foot 100 and the bladder is inflated. Bladder 9 is preferably of minimum size and volume consistent with its object of exerting compression pressure on the foot and therefore requires a minimum volume of pressurized air per pulse. It is seen that although the bladder 9 preferably occupies only the sole area of the foot 100, pressure and bladder expansion there causes the fabric enclosure around the foot to tighten and exert a compression force around most of the arch region 101 of the foot 100. Bladder 1 is primarily intended for pneumatic inflation, although other fluids could be substituted by those of ordinary skill in the art. In the first embodiment, bladder 1 is circular, roughly 3 to 5 inches in diameter. However, other shapes of bladders may be substituted while still employing many of the basic aspects of the invention. For instance, an elongate bladder can be provided in an orientation that encircles the arch region 101 of the foot 100 when it is properly applied. Figs. 21-23 show three alternative embodiments to the foot wrap 1A' shown in Fig. 3. Other variations and details are described in said Co-Pending U.S. Application and at least one other application referenced therein).

Referring to Figs. 21-23, each of the embodiments shown therein are constructed and used essentially using the same methods and materials as with the Fig. 3 embodiment, except as otherwise noted. Accordingly, like (or related) aspects of these various embodiments are numbered similarly. For instance, each embodiment has a strap that is positioned to be secured around the back of the leg of a person on which it is to be used, and the respective straps are numbered 7, 507,407 and 607, for Figs. 3, 21, 22, and 23 respectively. The differences from the embodiment of Fig. 3 and those of Figs. 21-23 will be fairly evident from a comparison of those Figures. Fig. 23 although showing a second tab 607 which does not extend substantially perpendicularly from the main portion shows an alternative positioning of the second tab relative to the main portion.

The principal difference between the Fig. 3 embodiment and the others is that each of those in Figs. 21-23 have some means for cushioning the leg strap 507, 407, and 607, respectively. That cushioning effect is believed to help inhibit the breakdown of skin that might otherwise be associated with straps 507, 407 and 607 -- particularly breakdown created by stress concentrations between the straps 507, 407 and 607 and the skin around the rearward ankle area of foot 100. Wrap 501 achieves the cushion by extending its inflatable bladder along the length of the strap 507. Wrap 601, on the other hand, achieves the cushioning effect by having a foam strap 607 that is inherently cushioned. Wrap 401 combines the approach of each by combining an inflatable extension of bladder 409 along the length of strap 407 together with an elongate patch of the foam material that is secured to strap 407 in an orientation coincident with the length of strap 407. The cushion foam used in the preferred embodiments of the embodiments shown in Figs 21-23 is commercially available in the U.S. under the trademark "Vel-Foam", although other cushion material could also be used.

Securing the foam to the rest of the foot wraps 401 and 601 may be accomplished either by a variety of conventional means, such as by stitching, glue, or heat-welded lamination. If stitching is to be used, the stitching is preferably done at a location of the wrap that is not inflatable, thereby avoiding leaks in the inflatable bladder. For instance, the Vel-Foam strap 607 is shown stitched to the remainder of wrap 601 with stitching 619 in a region 620. Region 620 is outside the weld 610 that defines bladder 609. With wrap 401 on the other hand, Vel-Foam patch 418 must be stitched around only three of its four edges (excluding edge 418') in order to avoid creating an air leak in bladder 409. Therefore it is preferable to glue patch 418 in place using conventional glues used by seamstresses for lasting application. Heat-welded dimples 417 and 617 are preferably spaced on the respective bladders 409 and 609 to minimize pillowing (excessive bulging) of the bladders 409 and 609 when they are inflated. Such dimples are best positioned at locations likely to overly bonier (or less squashable) areas of foot 100.

The shape of wrap 601 is unique, but it has a main bladder 609 that is also positioned under the arch of the foot 100. As with bladder 409, bladder 609 is elongate and is positionable transverse to the foot 100 to wrap around the foot 100. The positioning of strap 607 is oriented relative to the rest of wrap 609 to wrap higher around a patient's leg, as opposed to right around the ankle. Many other features of the embodiments shown in Figs. 21-23 will be evident to those of ordinary skill in the art in view of the other teachings herein, and in view of the prior art.

Referring again to the first embodiments, especially as shown in Figures 1, 6 & 7, fitting 11 (also called a "spicket" or "nozzle") is a tubular fluid connector having an elbow form to reduce its height profile. Its elbow shape also enables connection of a fluid hose (not shown) to the fitting 11 and helps minimize the possibility of kinking such a hose during use. Conventional hose connectors may be incorporated in the outermost end of fitting 11 to enable connection of such a hose, although a properly sized hose can also be connected merely by a friction fit with fitting 11. Fitting 11 is formed of a compatible heat-weldable material and has a base flange 12. This fitting is inserted through a hole punched in fabric sheet 2 so that flange 12 contacts the heat-weldable inner surface of fabric sheet 2 and is then welded fluid-tight to complete the bladder.

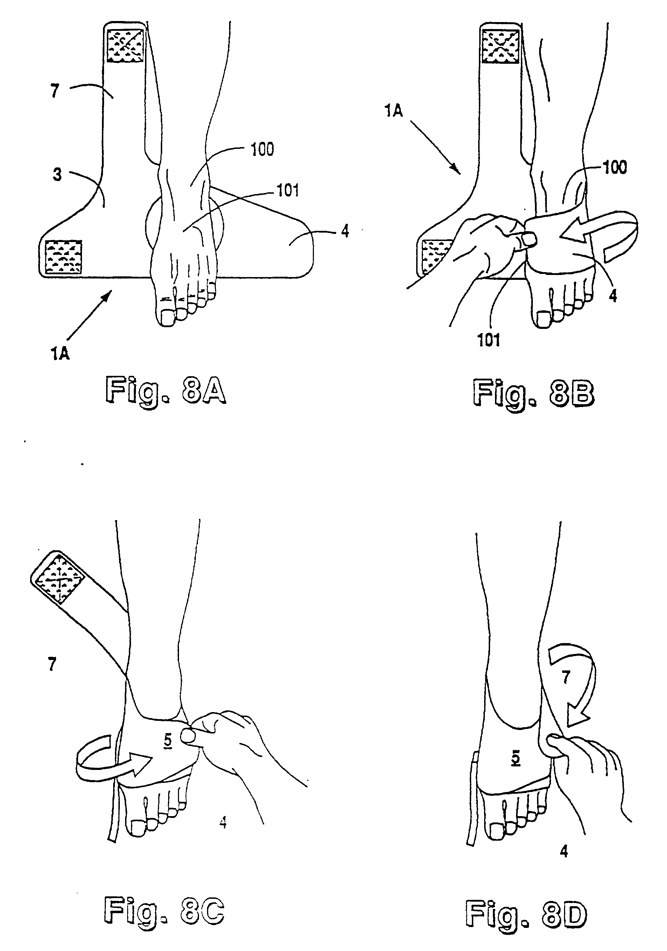

As mentioned, bladder 9 is formed in a main portion of foot wrap 1A', and tabs (or "extensions") 4, 5 and 7 extend generally away from the bladder 9. Tab 5 and a larger and longer extension 4 lie on opposite sides of the main portion that includes bladder 9, extending along the line "A-A". Extension or tab 7 of foot wrap 1A' lies substantially perpendicular to line "A-A" and is considerably longer and narrower than tab 5. In other preferred embodiments (such as wrap 1A in Figure 8), the tab 7 is more perpendicular than pictured in Figures 2-7. Edge 16 of tab 7 as shown in Figure 2 is aligned approximately tangent to the right hand (right in Figure 2) extremity of bladder 9. Hook patch 6 is sewn or welded at or near the distal end of tab 5 and is located, as shown in Figure 3, on the outer surface of inner sheet 3. The distal end of tab 7 is covered by a Velcro patch 8 in the same manner as tab 5 is covered with patch 6.

The outer perimeter 14 of the entire foot wrap 1A' is RF-welded to form a single composite sheet with the single tubular fitting 11 mounted therein. This preferred embodiment weighs less than 6 ounces and is approximately 38 centimeters in the direction of line "A-A" of Figure 2 by 39 1/2 centimeters in the perpendicular direction, which is in striking contrast to the large and complex foot wraps heretofore employed for this service. Other forms of connecting the sheets may be used, such as by stitching, although commensurate sacrifices of inventive aspects will be associated with such a change.

Referring to Figures 8A-8D, foot wrap 1A also stands out for its ease and simplicity of use. First place the foot wrap in the flat position shown in Figure 8A, inner sheet 3 in contact with the sole of the foot 100, heel parallel to tab 7 and extending in the same direction as tab 7. Then, as in Figure 8B, wrap tab 4 around the arch of the foot 100. Then wrap tab 5 over tab 4 as in Figure 8C, so tabs 4 & 5 overlap above the arch. Adjust the tightness of the fit to the degree desired and press the tip of tab 5 onto the outer surface of tab 4 so they form a secure connection. Then to completely secure the bladder 9 to the foot, draw tab 7 around the back of the foot (or heel) as shown in Figure 8D and pull it snug. Hooked tip 8 (shown in Figure 8A) is then pressed onto the outer surface of foot wrap 1A' where it overlaps on the side of the foot. The foot wrap is now locked in position until the fastenings are peeled open for removal of the foot wrap. This procedure can be accomplished in a few seconds, and removal requires only pulling of the two tabs 5 and 7.

The preferred position of the bladder 9 relative to the sole of the foot is illustrated in the drawings. Minor adjustments, if required, consist of loosening and repositioning one or both tabs 5 and 7 as necessary. The relative length of tabs 4 and 5 are not fixed but must meet the requirement of overlapping sufficiently to form a secure fastening when wrapped around a foot. Thus tab 4 may be shorter than tab 5, although the general proportion illustrated in Figures 1 - 8 are preferred. The foot wrap 1A' will fit a wide range of foot sizes without change in the application technique. Feet of very small persons may be fitted through the use of firm padding above the instep and behind the heel to simulate a larger foot while allowing the bladder to act directly against the sole of the foot.

The size and shape of the bladder 9 and foot wrap 1A' in relation to a foot may also be larger than shown, even enclosing the entire foot, while still appreciating many aspects of the invention. Although not included in the pictured embodiments in view of the objects of reducing complexity, size, weight and material cost, it would actually be ideal to provide a bladder which fully enclosed the foot or, perhaps more practically, extended to beneath the arches of the toes of the feet. However, larger capacity pumps and/or slower cycle times must be employed with such alternative embodiments in order to compensate for the bladders having larger volumes.

Nonetheless, without encompassing the entire foot, air can enter between the foot and the foot wrap from both the front and rear areas where the foot wrap wraps onto the foot. During the deflated phase of pumping, the fit is looser and air can more easily diffuse the approximately 3 inch distance required to completely cover the area of skin beneath said foot wrap. With proper materials as in the preferred embodiment, such diffusion is not critical though, as the material is vapor permeable - allowing moisture to vacate the skin surface during the deflation phase.

The soft inner surface of foot wrap 1A', which is also the outer surface of sheet 3, may be covered with a springy, open pile or other lining which promotes the entrance of air into the area between said foot wrap and the foot during the decompression phase. An alternative embodiment of the invention may use a non-vapor permeable sheet 3 having an outer surface with such air movement promoting characteristics.

This small, lightweight, inexpensive foot wrap 1A in itself is sure to fill an important need in modem medicine. Nonetheless, as will be evident from this description to those of ordinary skill in the art, many aspects of this invention may be appreciated with other compression devices, possibly in combination with other intermittent or sequential compression features or devices, and possibly employed on other parts of the body including the hand, the leg, the arm or combinations thereof ― naturally, with commensurate sacrifices of certain aspects of this present invention.

Referring again to Figure 1, foot pump housing 20 will be discussed briefly. Foot pump housing 20 is an injection molded plastic housing that comprises front housing plate 27 and back housing plate 26 fastened together using any conventional means such as screws. Front housing plate 27 and back housing plate 26, once connected, form handle 23 which facilitates carrying of pump unit 501. The preferred embodiment's pump unit 501 as a whole weighs roughly ten pounds, although the shape of housing 20 is ideal for units of any weight that can be safely hand-carried and supported on the foot board or headboard of a patient-supporting bed. The components inside the pump unit 501 are preferably arranged so the center of gravity for the pump unit 501 is roughly concentric with the volumetric centroid of the housing 20, although great care need not be taken in this as long as the center of gravity is appreciably below the height where the hooks 21 rest on the foot board 900. Such a location is preferred in order to distribute the unit's weight between its two mounting hooks 21A and 21B and to ensure the unit is not top-heavy, both for ease in carrying and stability in mounting. The shape of housing 20 and the relative positioning of hooks 21A and 21B naturally helps ensure the central center of gravity.

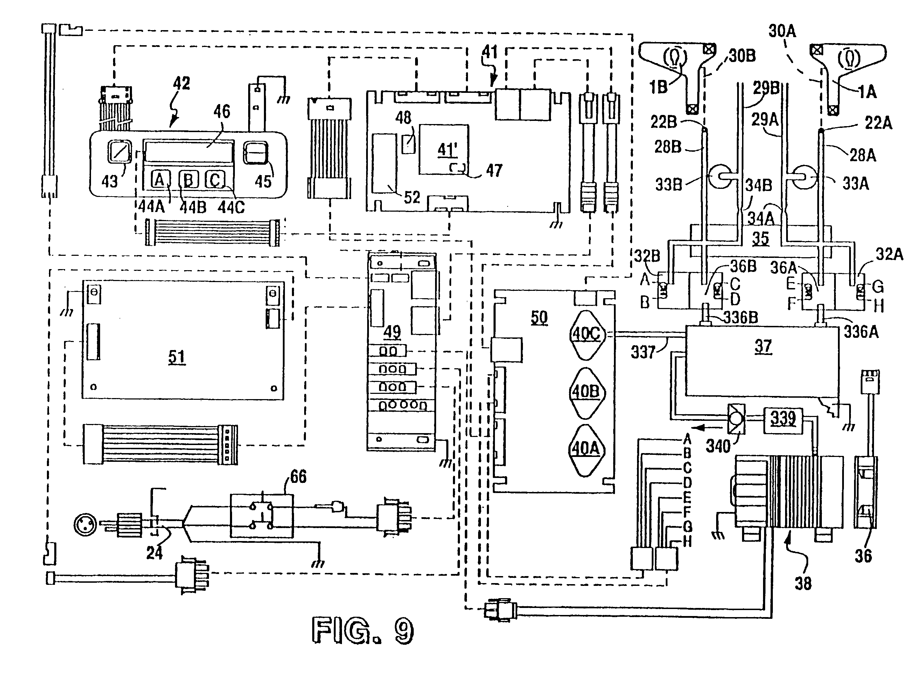

Referring still to Figure 1 and also to Figure 9, where more detailed features of system 500 are shown schematically, power switch 66 turns the pump unit 501 (and thus the system 500 as a whole) on and off. Power cord 24 is a conventional power cord for providing standard 115/120 volt, 60 Hz power to the pump unit 501. Preferably cord 24 is a hospital grade cord.

The links between pump unit 501 and foot wraps 1A and 1B are principally pneumatic. Right and left hose connectors 22A and 22B are the female members of conventional quick-disconnect air line connectors. Inlet connectors 22A & 22B are firmly secured to front plate 27 of housing 20 to provide a connection inlet from foot pump housing 20 to the respective air hoses 30A and 30B of right and left foot wraps 1A and 1B. The ends of hoses 30A and 30B that connect to housing 20 are adapted with integral male connectors 31A & 31B for easy mating with quick-disconnect inlets 22A & 22B. Connection and disconnection of connectors 31A and 31B can be done easily with one hand and little effort. The connections between hoses 30A & 30B and spickets 11A & 11B are relatively permanent connections for keeping the hoses 30 and wraps 1A & B integral.

Operator console 42 provides a user interface to the unit's microprocessor, allowing the user to either enter individual settings from a selection menu (discussed further herein) or select default settings. The primary processor of pump unit 501 is micro-controller 41, which essentially controls the operation of system 500. Operator console 42 basically comprises an LCD (liquid crystal display) assembly 46 formed in a membrane panel. LCD assembly 46 comprises a two line, twenty character alphanumeric liquid crystal display which displays programming instructions, status updates, and alarm messages to the operator. The membrane panel of console 42 has integral switches 43-45 therein, namely: "PULSE ON/OFF" switch 43; operator selection switches 44A, 44B, 44C respectively designated "A", "B" and "C"; and "OPTIONS" switch 45. As will be evident to those of ordinary skill in the art, other switch configurations and/or options may be substituted within the scope of this invention. Applicant, for instance, is presently considering elimination of the "PULSE ON/OFF" feature and changing the operation of switch 43 to silence any alarms, with appropriate designation of switch 43.

The operation of system 500 is primarily controlled by the primary processor of pump unit 501, which is micro-controller 41. Micro-controller 41 is a conventional type of control board. As will be evident to those of ordinary skill in the art the specific characters of micro-controller 41 are dictated by its intended operation, as described and evident further herein and in said Co-Pending U.S. Application (and other inclusions therein).

PULSE ON/OFF switch 43 both selects and de-selects an air pulse therapy mode for the system 500. If PULSE ON/OFF switch 43 is depressed to its ON state when the main power switch 66 is Off, the system 500 remains inoperative, but if PULSE ON/OFF switch 43 is depressed ON when the main power switch is ON, the controller of system 500 begins to deliver pneumatic pulses to the foot wraps 1A and 1B according to the currently set operating parameters. Such parameters are preset to default at power on to the last entered parameters, but they can be adjusted at any time thereafter by appropriate selection of the OPTIONS switch 45 and operator selection switches 44A-C when the main power switch 66 is ON, regardless whether switch 43 is actuated. Turning the PULSE ON/OFF switch OFF during operation will turn off compression/decompression cycling.

The remaining basic components of pump unit 501 are shown schematically in Figure 9, including without limitation: GPDU Power Distribution Board 49, power supply board 51, and air control board 50. On the pneumatic side, the unit 501 basically includes: compressor 38, compressor cooling fan 36, accumulator 37, two normally-closed inflation solenoid valves 36A & B, two normally-open deflation solenoid valves 32A & B, manifold 35, air hoses therebetween, and various other lesser components which will be clear from the drawings, particularly in view of the following more detailed descriptions of the system's operation.

The operating protocol of operator console 42 will now be described. When the main power switch for the system 500 is first turned ON, the micro-controller 41 (Figure 9) displays a query on the top line of LCD assembly 46 requesting the operator to indicate whether there is a new patient. The bottom line of LCD assembly 46 displays the choices "YES" or "NO" above operator selection switches 44A or 44B, respectively. Upon selection of "YES" by depressing switch 44A, the operator has 5 seconds to depress OPTIONS switch 45 (discussed herein); Otherwise, micro-controller 41 automatically stores the default values for the pump's settings (discussed herein) from ROM 52 into RAM 47 (see Figure 9). A selection of "NO" or no selection within 30 seconds causes micro-controller 41 to retrieve from the power down memory (discussed herein) the settings stored in memory when the unit was last powered off.

A depression of "YES" followed by a depression of OPTIONS switch 45 displays for user selection the first configuration (or "menu") option (see Table 1) on the top line of LCD assembly 46. Each subsequent depression of OPTIONS switch 45 sequentially displays a different configuration option for selection by the user (see Table 1). The bottom line of LCD assembly 46 simultaneously displays a selection of up to three choices for the configuration option displayed in the line immediately above. Those choices are displayed spaced across the second line of LCD assembly 46 with each choice centered above a corresponding operator selection (programming) switch 44A-44C. The operator enters a choice by depressing the operator selection switch 44A, 44B, or 44C that is directly beneath the appropriate display corresponding to the desired selection. Depression of operator selection switches 44A-44C appropriately signals micro-controller 41 to select the specified choice or query the operator for additional information. Therefore, by progressing through the display "option" menus and selecting the desired option parameters, the system operator may program all the options required for use during the operating session. However, if default values are selected, (note that values are always stored in power down memory) the default settings are: both feet selected, cycle time 20 seconds, hold time 3 seconds, and left and right pressure level 5. Table 1 discloses the menu selection options and the parameters available for use in the preferred embodiment of the present invention. Whenever the option selection process is complete, and whenever the "HOME" option is selected, the LCD display returns to one of three HOME displays as appropriate. The three HOME displays on the first line are: "Left and Right Pulse ON", "Left Only-Pulse ON", and "Right Only-Pulse ON". The second line of each HOME display has only one option - "(OFF)" - positioned above switch 44C. Selection of "(OFF)" deactivates the foot compression features of system 500 and the "ON" and "(OFF)" LCD displays are changed to "OFF" and "(ON)", respectively.

**TABLE 1**

| MENU SELECTION OPTIONS IN THE ORDER DISPLAYED | |
|---|---|
| Option | Description |
| Foot Pulse Select | Displays and allows the operator to select either one or both feet for pulse therapy. |
| | |
| Cycle Time Adjust? | "DECR INCR HOME" |
| | allows the operator to select the foot pump's cycle time; when both feet are selected, cycle time is total time required for foot wraps 1A and 1B (see Figure 9) to sequentially inflate, hold, and deflate; cycle times range from 10 to 60 seconds, user selectable in 10 second intervals. |
| | |
| Hold Time Adjust? | "DECR INCR HOME" |
| | permits the operator to adjust the amount of time that the pressure is maintained in the respective foot wrap after inflation occurs; selections are 1, 2, 3, 4, and 5 seconds. |
| | |
| Left Foot Pressure Adjust? | "DECR INCR HOME" |
| | permits the user to adjust the foot pressure level in the left foot wrap from levels 2 through 10. |
| | |
| Right Foot Pressure Adjust? | "DECR INCR HOME" |
| | permits the user to adjust the pressure level in the right foot wrap from levels 2 through 10. |
| | |
| View Timers? | "LEFT RIGHT HOME" |
| | allows the operator to view the foot pump's total therapy and operating times (Note that left and right foot therapy times are individually displayed). |
| | |
| For Service Use Only | used by maintenance personnel to calibrate the foot pump. |

In actual use, the pressure levels corresponding to the range of levels 2 to 10 in the preferred embodiment are roughly 25-200mm Hz, although larger bladders (such as that shown in Fig. 23 may tend to lower the range of pressures.

For the purposes of disclosure, the timing and pressure setting ranges along with the system default settings of the preferred embodiment of the present invention have been described. The variability of such settings is highly beneficial. However, one of ordinary skill in the art will readily recognize that other ranges and default settings could be substituted.

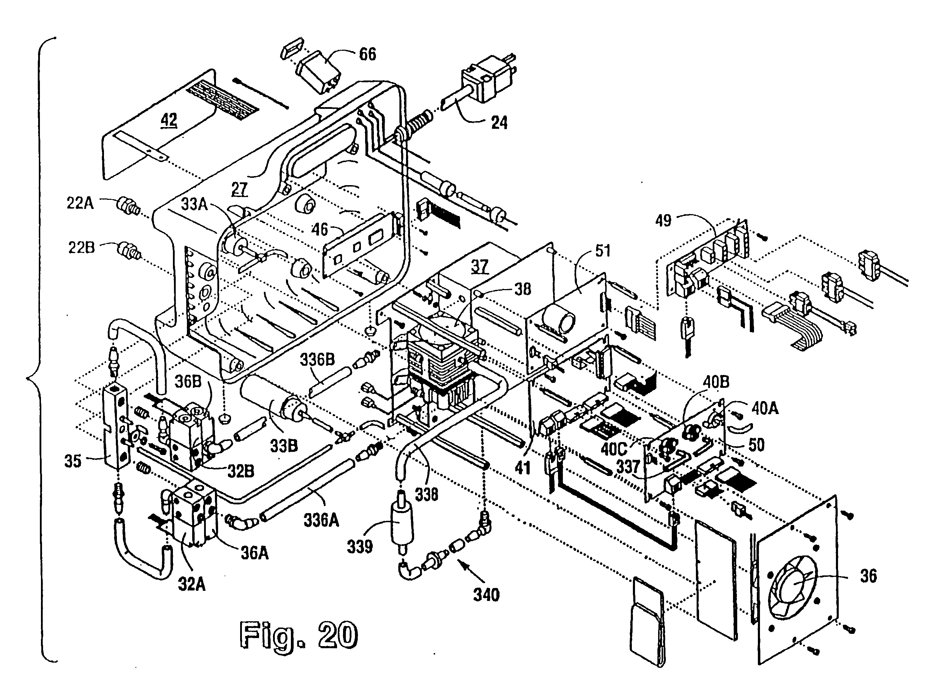

Referring to both Figures 9 and 20, the preferred system component configuration and operation will be discussed in more detail. Foot wraps 1A and 1B, designed to be securely fastened to a human foot, are constructed from two sheets of fabric sewn or welded together to form an inflatable pocket. A complete description of foot wraps 1A and 1B are provided in said Co-Pending U.S. Application. The disclosures included in that application are incorporated herein by this and previous references herein, as though set forth in their entirety. Alternative compression devices which are known in the art (such as compression sleeves or gloves) also could be used to appreciate many aspects of the invention.

Power cord 24 delivers the overall system power supplied from the standard 115V/120V, 60 Hz source to GPDU power distribution board 49 and, in turn, to Power supply board 51. Power supply 51 converts the standard 115V/120V AC to 12V DC and supplies the 12V DC back to power distribution board 49. Power distribution board 49 distributes the 115V/120V AC and the 12V DC to each component according to the electrical lines pictured in Figure 9 and the particular component's power requirement.

Compressor 38 provides the compressed air which is necessary to inflate the inflatable bladders formed in foot wraps 1A and 1B. Accumulator 37 stores the compressed air generated by compressor 38 before delivery to foot wraps 1A and 1B. Micro-controller 41 polls PULSE ON/OFF switch 43 to determine when compressor 38 should be turned on or off. In the presently preferred embodiment, the compressor is always running....When it reaches its maximum pressure, it simply turns the air without pumping it. Nonetheless, the system 500 could be modified such that the pressure requirements of the system factor in determining the operation of solenoid valves 32 and 36. Compressor 38 is turned on and off with the system so as a whole by actuation of switch 66. Fan 36 also operates to continually supply a flow of air over compressor 38 to prevent it from overheating.

In operation of the preferred embodiment, the operator may select either the right foot, left foot or both feet for foot pulse therapy. As described before, such a selection is enabled by micro-controller 41 and console 46. If both feet are selected for therapy, foot wraps 1A and 1B alternately inflate and deflate to provide pressure against a patient's foot. However, for the purposes of disclosure, only operation of the right foot wrap will be described. However, it is to be understood that the left foot wrap operates in the same manner. The numeral designations "A", however, are sometimes left off reference numerals to enable easy reading on either the left or right foot wrap systems. Right solenoid valve 36 opens to inflate right foot wrap 1A by allowing air pressure from accumulator 37 to be directed into right manifold 35. Right manifold 35 simultaneously directs that air pressure to: right foot wrap 1A via hose 30A, quick connect 31A, and external hose 30A (shown best in Fig. 1); right vent solenoid valve 32; and right foot wrap pressure sensor 40A via sensor hose 29a, which has restrictor 34A and expansion tank 33A along its course. While foot wrap 1A is being inflated (and thereafter through the hold time) to the selected or default pressure, right solenoid valve 36 is held closed, thereby maintaining the air pressure in foot wrap 1A. After the operator-selected or default hold time elapses, right vent solenoid valve 32 opens, discharging the air from foot wrap 1A. The above cycle then repeats after the cycle time (i.e. the time between inflations) elapses.

The controller divides solenoid operation into four modes, as shown below:

| | Printlets | Inflate | Hold | Deflate |
|---|---|---|---|---|
| INFLATE | OFF | ON | OFF | OFF |
| DEFLATE | ON | ON | ON | OFF |

An initial surge of compressed air occurs when right solenoid valve 36 is first opened. If right foot wrap pressure sensor 40A measures the initial surge of air, an erroneous pressure measurement occur. That is, right pressure sensor 40A would measure the pressure level as being too high. To prevent right pressure sensor 40A from measuring the pressure surge, the air flow path to right pressure sensor 40A contains right restrictor 34A and right expansion tank 33A. Right restrictor 34A restricts the flow of compressed air into right expansion tank 33A, thus decreasing the flow rate of the compressed air. Right expansion tank 33A provides an increased volume for expansion of the compressed air. As the initial air surge expands into right expansion tank 33A, the air pressure decreases. Therefore, when right foot wrap pressure sensor 40A measures the initial surge of air pressure, that measurement approximates the actual pressure being delivered from compressor 38 via accumulator 37 rather than the initial surge. Hence, the combination of restrictor 34A and tank 33A (and to an extent, each of them individually) serve as means for eliminating spikes in the pressure feedback sensed by sensor 40A.

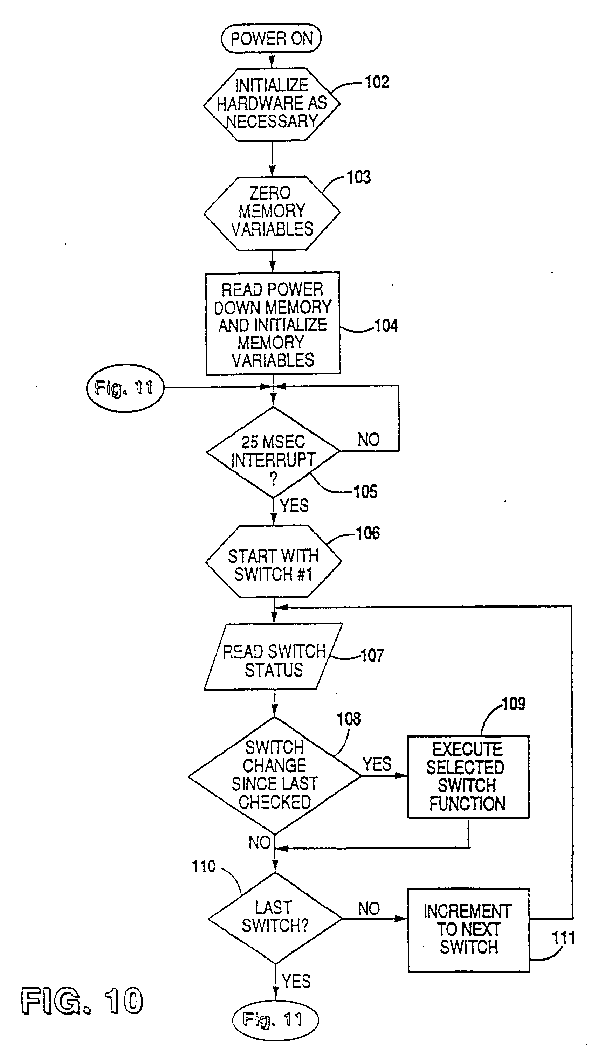

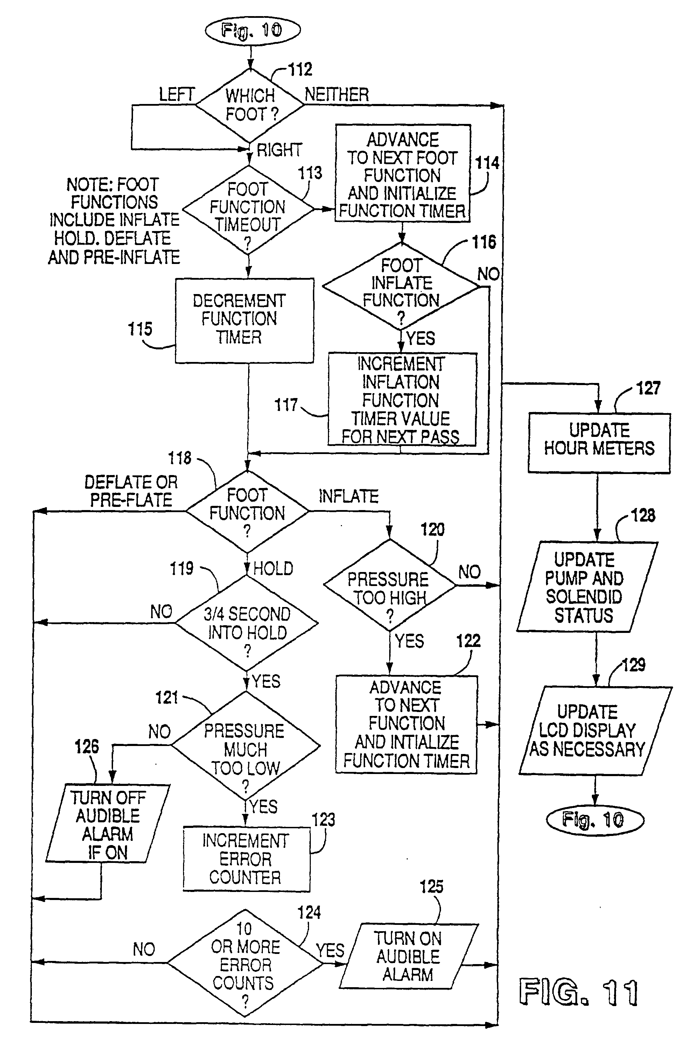

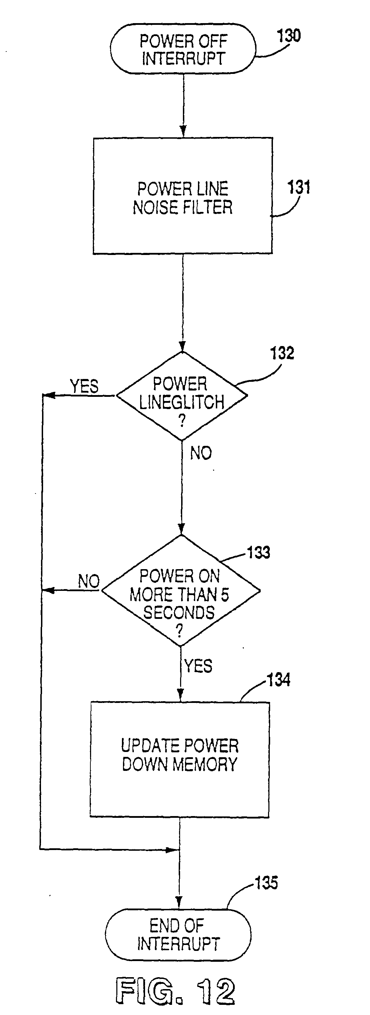

Right foot wrap pressure sensor 40A, a conventional pressure transducer in the preferred embodiment, converts the pressure delivered to right foot wrap 1A from accumulator 37 into an electrical signal representative of that pressure. An analog-to-digital (A/D) converter (not shown) converts that electrical signal to a digital signal readable by micro-controller 41. Micro-controller 41 processes that pressure signal to control right solenoid inflate valve 36 and right vent solenoid valve 32. Control of the solenoid inflate valves and vent solenoid valves by micro-controller 41 will be discussed herein with reference to the flow charts shown in Figures 10-12. (Note: Pressure sensor and amplifiers located on air control bond 50 send pressure defts to micro-controller board 41. Micro-controller board 41 then [after processing] sends solenoid control information back to solenoid drives circuits which are also located on board 41. These drives circuits control the solenoids.

Pressure transducer 40C is identical to transducers 40A and 40B but is provided to sense the pressure in accumulator tank 37 - not foot wraps 1A or 1B. Specifically, the air port of sensor 40C is in direct fluid communication with tank 37 through sealed line 337. The ultimate function of sensor 40C is to sense the drop in pressure after each opening operation of valves 36A or 36B, thereby giving controller 41' an indication of whether air is flowing freely out of tank 37. Micro-controller 41' uses that pressure reading for diagnostic display and audible alarm purposes. Micro-controller 41' determines if the pressure is too low, based on a digital signal from the A/D converter associated with the appropriate pressure transducer 40. Micro-controller 41, in combination with the pressure transducer 40C, thus, serves as a means for detecting errors in the operation of system 500 in its preferred embodiment - specifically errors due to obstruction in air flow to (and thus from, as well) the foot wraps 1A and 1B. If either line 28A or 28B are kinked, the pressure in tank 37 will not drop after opening the respective valve 36A or 36B. If controller 41' determines the pressure drop sensed by sensor 40C does not exceed a threshhold level corresponding to roughly 5mm Hg for five consecutive cycles, the controller 41' concludes there is a kink in line 28A or 28B and signals an audible alarm and visual alarm. In more detail, if the pressure drop after valve opening is too low, micro-controller 41 increments an error counter. Micro-controller 41 determines if the error counter is greater than or equal to a value of five. Then, if the error counter is greater than or equal to five, micro-controller 41 turns on an audible alarm, although the pump unit 501 continues to operate as before. As will be apparent to those of skill in the art, A/D converters (not shown) are associated with each sensor 40A-40C to enable interface with controller 41'.

Referring to the flow chart shown in Figures 10-12, micro-controller 41 control of the preferred embodiment of the present invention will be described. Preferably, micro-controller 41 is a conventional micro-controller such as Signetic's 80C552 controller, which has one RAM chip 47 (along with other conventional components) integral therewith. After the power is turned by depressing switch 66 on Fig. 9 and 26 on Fig. 1, micro-controller 41 actuates an initialization signal to its hardware components including the circuits controlling the pump, solenoids and display. In step 103, micro-controller 41 places zeros in the variables contained in random access memory (RAM) 47. When the foot pump is powered down, its present configuration settings, referred to as power down settings, are stored in EPROM 48 and may be used as default settings for the next power up. Accordingly, in step 104, the power down settings stored in EEPROM 48 are read and stored in RAM 47.

Sequential polling of PULSE ON/OFF switch 43, operator selection switches 44A, 44B, and 44C, and OPTIONS switch 45 occurs every 25 milliseconds, as shown in steps 105-111. Step 106 informs micro-controller 41 to start its polling with switch 1. In step 107, micro-controller 41 stores the state of the polled switch in RAM 47 and in step 108 compares that state with its previous state. If the switch has changed states, micro-controller 41 advances to step 109 and executes the selected switch function and stores the operator setting or default setting retrieved from ROM 52 (actually a conventional EEPROM in the preferred embodiment) into RAM 47. Micro-controller 41 then advances to step 110. Alternatively, micro-controller 41 advances directly to step 110 if the switch did not change state. In step 110, micro-controller 41 determines if the last switch has been polled. If the last switch has not been polled, micro-controller 41 increments to the next switch in step 111 and returns to step 107 where it reads the state of that switch. Once all the switches have been polled, micro-controller 41 advances to step 112.

In step 112, micro-controller 41 reads the settings from status register located in a RAM therein to determine which foot if either will be processed for the current cycle. (RAM is this product that is located inside the micro-controller integrated circuit 41'). If Pulse is switched OFF, micro-controller 41 advances to step 127 and updates the operating time meter, which maintains total operating time. In step 128, micro-controller 41 actuates signals to both power distribution board 49 and pressure control board 50 to control compressor 38 and the inflate solenoid the vent solenoid valves, respectively. In response, power distribution board 49 turns compressor 38 on or off, and pressure control 50 energizes or de-energizes as necessary the inflate solenoid valves or vent solenoid valves. In step 129, micro-controller 41 refreshes the present display of LCD assembly 46 as necessary. After updating the display on LCD assembly 46, micro-controller 41 returns to step 105 to continue the present foot function mode. This loop is respected every 25 milliseconds.

The four foot function modes will now be discussed. This path is followed anytime that PULSE is switched ON. Micro-controller 41 sequentially and repeatedly advances through the following foot function modes: inflate to hold, hold to deflate, deflate to pre-inflate, and pre-inflate back to inflate. During an inflate cycle, the inflate solenoid valve corresponding to the foot wrap that is to be inflated opens, allowing air pressure to fill the inflatable bladder. After each inflate cycle, the opened solenoid valve closes, thus maintaining the air pressure in the foot wrap during the hold mode. In the deflate mode, the vent solenoid valve corresponding to the inflated foot wrap opens releasing the air pressure. The pre-inflate mode allows the opened vent solenoid valve to close, and the vent solenoid to close before the foot pump returns to the inflate mode.

The above cycle continuously repeats during foot pump operation. However, when the pump unit 501 is initially turned on, the beginning inflate modes operate under a ramping function. A ramping feature is beneficial because the pressure sensation delivered to a patient's foot during the first few inflate modes may startle the patient. Accordingly, the ramping feature reduces patient alarm by allowing the patient to gradually become acclimated to the pressure pulses delivered to the patient's foot. Upon system start-up, the initial pressure in the first inflate mode is low because the solenoid valves are only briefly energized (25 milliseconds in the preferred embodiment). During each subsequent inflate cycle, the solenoid valve energization time increases by 25 milliseconds until that energization time reaches the operator selected or default pressure level, and the foot pump is delivering the desired pressure to the patient's foot.

Again referring to step 112, if the left or right foot is selected, micro-controller 41 advances to step 113 and reads the value of the foot function counter. The value stored in the foot function counter represents the time remaining for the foot function in progress. Foot functions include inflate, hold, deflate and preinflate. Each foot function counter value is determined on the basis that micro-controller 41 requires 25 milliseconds (in the preferred embodiment) to execute steps 105-129, thereby completing one processing loop, which includes a foot function counter decrement (step 115). For example, if the user selects a one second hold time, micro-controller 41 places a value of 40 in the foot function counter. Micro-controller 41 enters that value into the foot function counter at the beginning of a hold function because 40 loops through steps 105-129 equals one second (40 loops times 25 milliseconds equals 1000 milliseconds or one second). Therefore, as micro-controller 41 decrements the hold mode foot function counter during each pass through step 115, the inflate solenoid valves and vent solenoid valves will remain closed for one second.

The hold, deflate, and pre-inflate modes have fixed foot function counter values corresponding to each operator or default time selection as calculated above. However, because of the pressure ramping feature, the inflate mode foot function counter begins at 1 (corresponding to a real time of 25 milliseconds) and is incremented by 1 during each subsequent inflate mode until the desired pressure is reached. Thus, the solenoid valves remain open an additional 25 milliseconds through each subsequent inflate cycle. Once the desired pressure is achieved, micro-controller 41 no longer increments the inflate mode foot function counter.

Although for the purposes of a preferred embodiment, micro-controller 41 requires 25 milliseconds to perform the functions disclosed in steps 105-129, one of ordinary skill in the art will readily recognize that any delay time could be properly substituted.

In step 113, if the foot function counter is not 0, micro-controller 41 advances to step 115 and decrements the foot function counter by 1. Micro-controller 41 then advances to step 118. If the foot function counter is 0, micro-controller 41 has completed a foot function inflate, hold, deflate or pre-inflate. Accordingly, micro-controller 41 advances to step 114 and increments to the next foot function and places the value corresponding to that foot function into the foot function counter. Next, in step 116, micro-controller 41 determines whether the present foot function is in the inflate mode. If the foot function is in the inflate mode, micro-controller 41 advances to step 117 and increases the previous foot function inflate count by 1 (corresponding to 25 milliseconds) for use during the next inflate cycle. However, if the inflate mode has reached the user selected or default pressure as determined in step 120 (discussed herein), micro-controller 41 will not increment the foot function counter. If the foot function is not in the inflate mode, micro-controller 41 advances directly to step 118.

In step 118, micro-controller 41 determines whether the foot function is in the deflate, pre-inflate, hold, or inflate mode. If in step 118, the foot function was in the inflate mode, micro-controller reads a pressure signal from the respecting pressure sensor and advances to step 120. In step 120, micro-controller 41 determines if the pressure in the foot wrap is too high. That is, micro-controller 41 determines if the foot wrap pressure has exceeded the operator selected or default pressure. If the measured pressure is below the operator selected or default pressure, micro-controller 41 advances to step 127 and updates the operating time meter. In step 128, if the solenoid valve corresponding to the foot wrap to be inflated is de-energized, micro-controller energizes it via pressure control 50. However, if the solenoid valve is already energized, micro-controller 41 rewrites the energization signal to pressure control 50. (Note that the vent solenoid status is updated as well.) Micro-controller 41 then updates LCD assembly 46 and returns to step 105 to continue the present foot function.

However, if, in step 120, micro-controller 41 determines that the actual pressure is above the desired pressure, it advances to step 122. In step 122, micro-controller 41 increments to the next foot function (hold in this case) and places the foot function counter value corresponding to that foot function in the foot function counter. Micro-controller 41 then updates the operating time meter in step 127. In step 128, micro-controller de-energizes, via pressure control 50, the inflate solenoid valve that had been energized during the inflate mode. The vent solenoid status is updated as well. Next, micro-controller 41 updates LCD assembly 46 and returns to step 105 for execution of the next foot function mode.

Micro-controller 41 is provided with two methods in the inflate mode to stop compressed air delivery to the foot wraps. First, micro-controller 41 monitors the pressure developed in the foot wrap during inflation, and if that pressure exceeds the operator selected or default pressure value, micro-controller 41 immediately increments to the next foot function and closes the energized solenoid valve (steps 120, 122 and 128). Alternatively, if the air pressure ever exceeds the desired pressure, micro-controller 41 delivers compressed air for the incrementing interval defined in the foot function counter. That is, micro-controller 41 allows inflation of the foot wrap until the inflate mode foot function counter is decremented to 0. Once the foot function counter reaches 0, micro-controller 41 increments to the next foot function and de-energizes the energized solenoid valve, thereby stopping air pressure delivery to the foot wrap. Thus, micro-controller 41 stops compressed air delivery to the foot wrap when either the inflation pressure exceeds the user specified or default value, or the inflate mode foot function counter reaches 0, whichever occurs first. (As noted earlier in this document, the inflate time valve is romped up in 25 millisecond intervals.)

Again referring to step 118, if the foot function is in the hold mode, micro-controller 41 advances to step 119 and determines whether at least 3/4 of a second of the hold time has elapsed. If at least 3/4 of a second of the hold time has not elapsed, micro-controller 41 advances to step 127 and updates the operating time meter. In step 128, if the inflate mode foot function counter was completely decremented to 0, micro-controller 41 will de-energize via pressure control 50 the solenoid valve energized during the preceding inflate mode. However, if the inflate mode was ended because the air pressure in the foot wrap became too high, the energized solenoid will already have been de-energized, and micro-controller 41 will only rewrite the de-energization signal. Micro-controller 41 then updates the display on LCD assembly 46 in step 129 and advances to step 105 to continue the present foot function mode.

If the elapsed hold time is equal to 3/4 of a second, micro-controller 41 reads the pressure value measured by the respective pressure sensor and advances to step 121. Step 119 provides a 3/4 of a second delay before pressure measurement to permit the air pressure in the foot wrap to settle so that the pressure sensor makes an accurate pressure reading. Micro-controller 41 uses that pressure reading for diagnostic display and audible alarm purposes. In step 121, micro-controller 41 determines if the pressure is too low, based on a digital signal from the A/D converter associated with the appropriate pressure transducer 40. Micro-controller 41, in combination with the pressure transducer 40, thus, serves as a means for detecting errors in the operation of system 500 in its preferred embodiment. If the pressure is too low, micro-controller 41 advances to step 123 and increments an error counter. In step 124, micro-controller 41 determines if the error counter is greater than or equal to a value of 5. If the error counter is greater than or equals to 5, micro-controller 41 turns on an audible alarm in step 125 and advances to step 127. Although the audible alarm sounds, the pump unit 501 continues to operate, and no changes are made. In step 127, micro-controller 41 updates the operating time meter. In step 128, micro-controller rewrites the present actuation signals to power distribution board 49 and pressure control board 50. In step 129, micro-controller 41 updates the display as necessary on LCD assembly 46 and returns to step 105 to continue the present foot function mode. However, if the error counter is below 5, micro-controller 41 advances directly to step 127 without turning on the audible alarm. In steps 127-129, micro-controller 41 updates the operating time meter, rewrites the actuation signals to power distribution board 49 and pressure control board 50, and updates the display as necessary on LCD assembly 46. Micro-controller 41 then advances to step 105 to continue the present foot function.

Again referring to step 121, if the measured pressure is not too low, micro-controller 41 advances to step 126, turns off the audible alarm (if it was on), and sets the error counter to 0. Micro-controller 41 then advances to steps 127. In steps 127-129, micro-controller 41 updates the operating time meter, rewrites the actuation signals to power distribution board 49 and pressure control board 50, and updates the display as necessary on LCD assembly 46. Micro-controller 41 then advances to step 105 to continue the present foot function.

Referring again to step 118, if the foot function is in the deflate or pre-inflate mode, micro-controller 41 advances directly to step 127 to update the operating time meter. In step 128, micro-controller 41 changes the actuation signal to pressure control board 50 to either energize or de-energize the vent and inflate solenoid valves dependent upon whether the foot function mode is deflate or pre-inflate. For example, if the foot function is in the deflate mode, the vent solenoid valve will open and the inflate solenoid valve will be closed to allow the deflation of the inflated foot wrap. However, if the foot function is in the pre-inflate mode, the vent solenoid valve will be closed in preparation for the next inflate foot function mode. Also in step 128, micro-controller 41, via power distribution board 49, stops, starts or continues the delivery of power to compressor 38. After micro-controller 41 updates LCD assembly 46 in step 129, it advances to step 105 where the loop is repeated every 25 milliseconds.

Operation of the power down memory of the preferred embodiment of the present invention will be described in reference to the flowchart shown in Figure 4. In step 130, if the power is turned off or there is a power line transient (e.g. a power surge or noise), micro-controller 41 executes the power off interrupt. In step 131, micro-controller 41 executes a software filter loop to reduce susceptibility to power line noise transients. In step 132, micro-controller 41 determines if the power has been turned off or if the power line merely experienced a transient. If the power line experienced a transient, the interrupt routine is exited and the pump unit 501 continues to operate normally. If micro-controller 41 determines that AC power has been turned off, micro-controller 41 advances to step 133 and determines if the power has been on for more then 3 seconds. If the power has been on for more than 3 seconds, micro-controller 41 advances to step 134 and writes date into EEPROM power down memory 48 values representing the last operator entered foot function settings. Micro-controller 41 then advances to step 135 and exits the power down routine. However, if the power has not been on more then 3 seconds, micro-controller advances to step 135 and exits the power down routine.

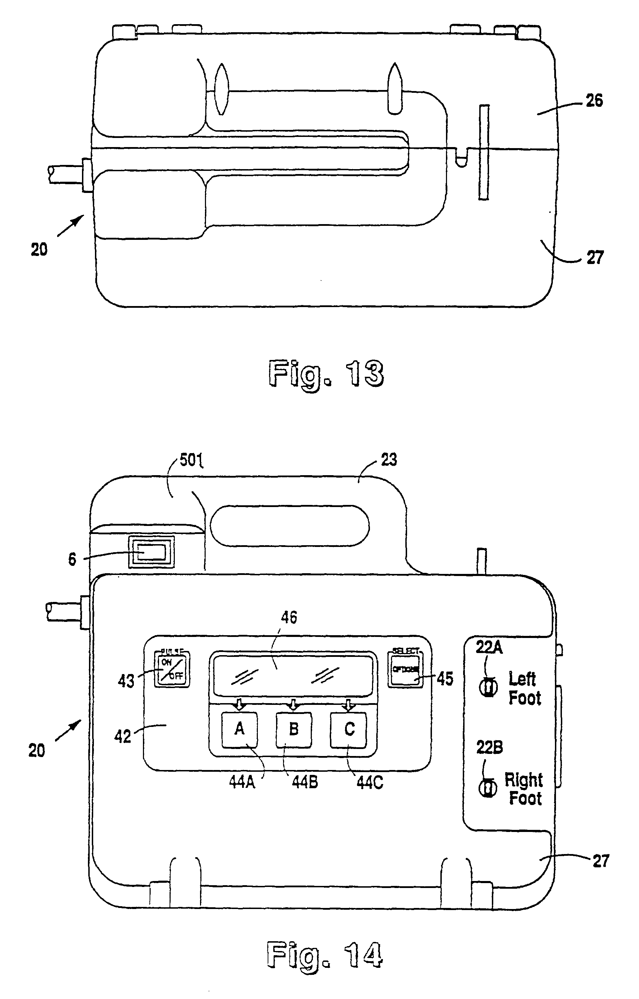

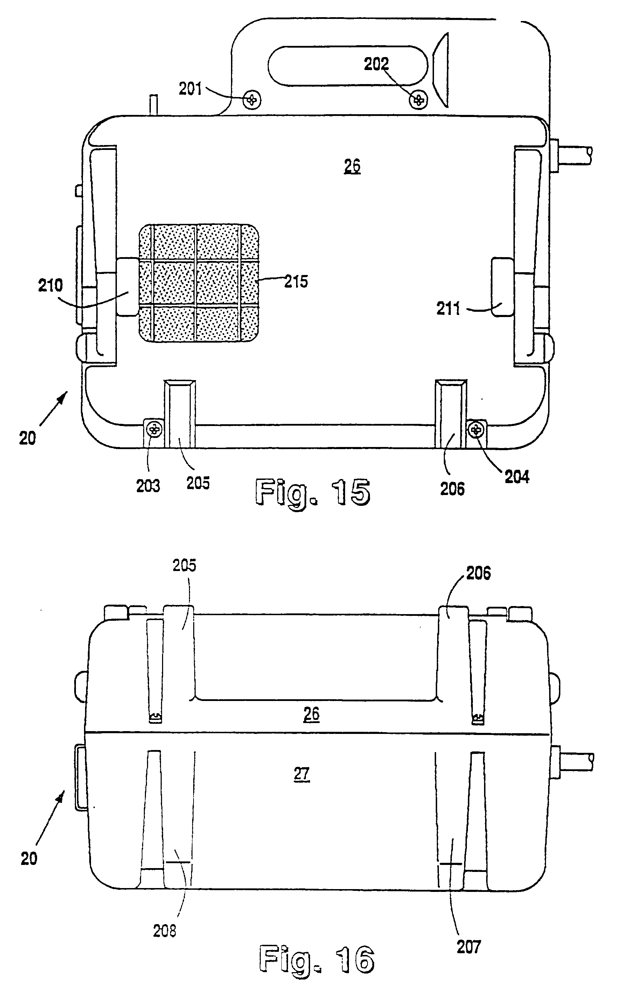

Figures 13-18 show the general shape of the pump housing 20 which has also been described with reference to Figure 1. Note though that the details of console 42 and connectors 22A and 22B have been omitted in Figure 14 to focus attention on the shape of housing 20. Screws 201-204 (shown in Figure 15) are common screws which secure front plate 26 to rear plate 27. Of course, other conventional connectors could be used instead. Spacers 205-208 on the underside of housing 20 allow for level placement of the pump unit 501 on a flat surface, whereas spacers 210 and 211 ensure free flow of air into the pump unit's air intake 215, even when the unit is mounted snugly against a foot board of a hospital bed.

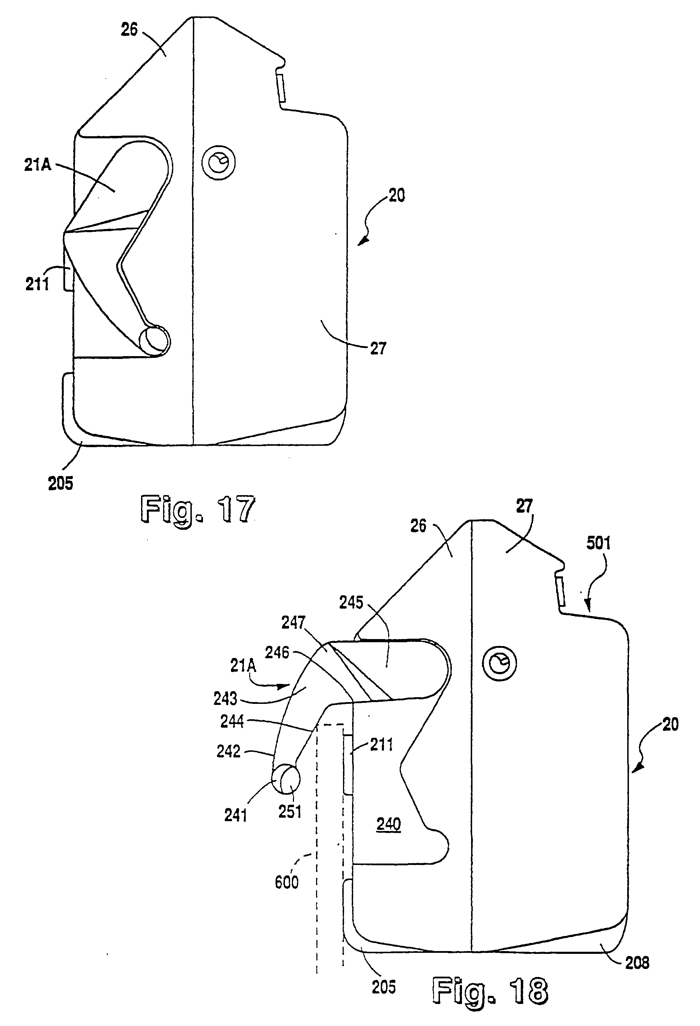

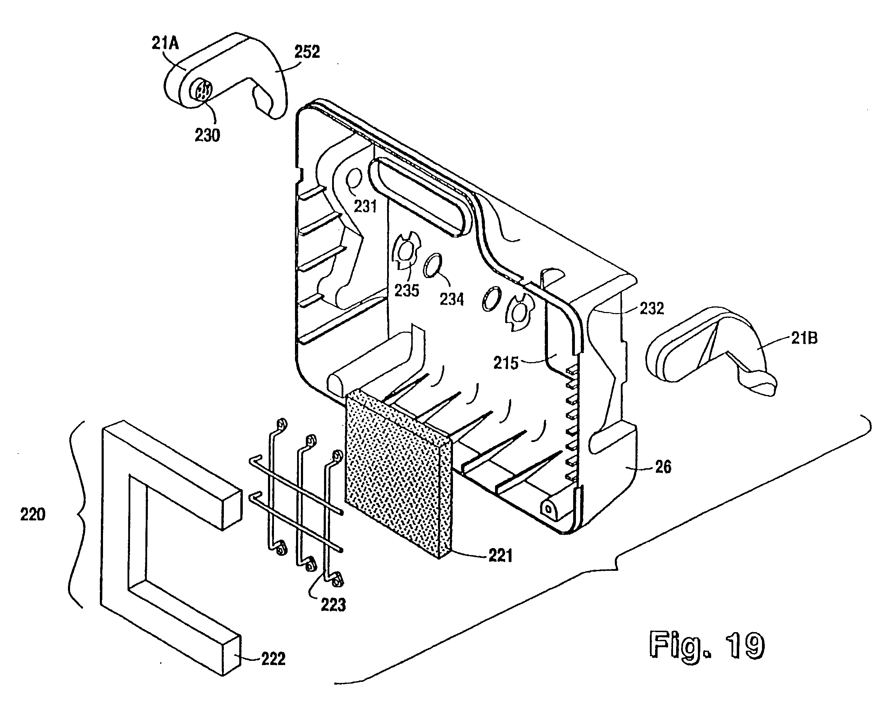

Assembly of the back plate 26 is best understood with reference to Figure 19. As shown therein, air intake filter assembly 220 is mounted to the inside surface of back plate 26. Assembly 220 basically includes a primary filter 221 and a secondary filter 222 together with mounting bracket 223. Primary filter 221 is fairly porous to allow free intake of air while secondary filter 222 is positioned to surround the internally-exposed edges of primary filter 221 to ensure that all intake air is adequately filtered even if it circumvents primary filter 221. Bracket 223 is rigidly secured to the back plate 26 in a manner that cages primary filter 221 in place over intake 215. Secondary filter 222 is glued in place afterwards. The location of intake 215 is ideal as it is partially shielded from liquids and objects that might block the intake when housing 20 is operatively mounted on a foot board of a hospital bed. Spacers 210 and 211 preferably provide a 3/8 inch separation between the foot board 600 (shown in phantom line in Figure 18) and intake 215 to ensure free air flow therein.

Retractable mounting hooks 21A and 21B are shaped as shown best in Figures 17 and 18. Hooks 21A and 21B are similar to each other and are symmetrically mounted in a pivotable manner to plate 26. The pivotable mounting is best shown in Figure 19, with reference to hook 21A. As shown therein, hook 21A is pivotably mounted by means of an integral shank 230 that fits snugly through a hole 231 in an irregular socket 240 (numbered in Figure 18) formed in plate 26. Hole 231 is substantially coaxial with hole 232 so that the hooks 21A & B pivot in parallel planes that are perpendicular to back plate 26 in the preferred embodiment. Once inserted through hole 231, shank 230 is pivotally secured therein by a locking push ring 234. A spring washer 235 is positioned on the shank 230 to spring bias the shank through the hole in the same manner as a Bellview washer. Although not clear in Figure 19, portions of spring washer 235 are bent in a manner that provides such a spring action. The locking push ring 234 is appropriately sized and flared around its inner circumference in a conventional manner so that it can be pushed onto shank 231 but the flare prevents it from sliding back off the shank 231. Thus ring 234 provides a permanent pivotal connection for hook 21A.

Although not shown very well in the drawings, it is preferred that slight tongue and groove reliefs are provided on the mating surfaces between the hook 21A and plate 26 so that hook 21A tends to click into place in either its fully retracted or fully extended positions. The spring bias of washer 235 helps provide such a clicking action as it tends to retain hook 21A in its clicked position. Figure 17 shows hook 21A in its fully retracted position, and Figure 18 shows hook 21 B in its fully extended position. Socket 240 is shaped to neatly receive hook 21A when it travels from its fully extended to its fully retracted positions.

The innovative shape of hook 21A is best appreciated with reference to Figure 18. Progressing from shank 230 outward, portions of hook 21A are referred to as shoulder 245, elbow 247, forearm 243, and knob 241, all formed as an integral member which is generally progressively thinner toward knob 241. The inner face 252 of hook 21A approximates a planar surface (except for the protrusion of shank 230) so that hook 21A pivots freely into socket 240. Knob 241, on the other hand, protrudes outward in a direction opposite shank 230 to roughly twice the thickness of forearm 243. Knob 241 is cylindrical in shape with its leading outer corner truncated and rounded to form a smooth finger pad 251. Finger pad 251 enables manual pivoting of hook 21A from its fully retracted to its fully extended position. The point of juncture between knob 241 and forearm 243 is referred to as wrist 242.

The forwardmost edges 244 and 246 of forearm 243 and shoulder 245 are substantially planar sections which are parallel to axis 250 of shank 230 and, therefore, appear linear in Figures 17 and 18. Although fillets are provided at wrist 242 to reduce stress concentrations, the plane of edge 244 is roughly perpendicular to the cylindrical surface of knob 241. Knob 241 thus forms a protruding lip at the distal end of forearm 243, and this lip helps retain pump unit 501 on foot board 600 even if it is knocked outward from the foot board.

Because standard foot boards range from 1/2 to 1 1/2 inches in width, it may also be helpful to consider some of the dimensions of hook 21A. The pivot axis 250 for the hook 21A is preferably as high as possible on housing 20 and should be at least high enough to ensure the center or gravity of pump unit 501 is lower than the center of distal knob 241, to help ensure against tipping. To accommodate a range of foot board sizes, the distance between the crease 248 of elbow 247 and the planar surface 26' of back plate 26 (when hook 21A is fully extended) is 7/8 inches, thereby snugly receiving the smallest of standard foot boards while leaving the 3/8 inch space provided by spacers 210 & 211. At the opposite extreme, the forwardmost edge of knob 241 is 1 7/8 inches from surface 26' (when fully extended), thereby snugly accommodating the widest of standard hospital foot boards. Moreover, for any foot boards in the range of standard sizes, the weight of pump unit 501 tends to pull surface 26' downward and thus closer to foot board 600 due to the constant slope of edge 244, until each of spacers 210 & 211 engage the foot board 600. Referring to angle alpha (Figure 18) the slope is such that alpha is an obtuse angle less than 135 degrees. Preferably, alpha is roughly 115 degrees. With such construction, housing 20 is adapted for snug, secure and neat mounting on hospital bed foot boards 600 in the full range of standard sizes.

Although the present invention has been described in terms of the foregoing preferred embodiments, such embodiments are merely exemplary of the present invention. As will be apparent to those of ordinary skill in the art, many other alternatives, equivalents, modifications, objects, substitutions, variations and the like, of varying degrees, will fall within the scope of the present invention. For instance, the number, specifications and locations of various components or the sequence, number, and complexity of various steps can generally be modified to some degree while still serving substantially the same purposes of the present invention. Accordingly, nothing in the foregoing detailed descriptions limits the scope of the present invention in any respect, but rather that scope is defined instead only by the claims which follow, construed as broadly as possible.

## Claims

1. A medical apparatus (500) for cyclically applying pressure to a foot (100) comprising:
a flexible fabric foot wrap (1A,1B,1A', 401,501,601) formed from two sheets (3,4) and having an inflatable bladder (9A,9B,409,509,609) therein;
a connector (11,411,511) opening into the inflatable bladder and suitable for connection to a source of pressurized fluid;
said foot wrap having a main portion including a first tab (5,405,505,605) formed from at least one of said two sheets the main portion being adapted for releasably securing the device to a human foot (100) about an arch thereof, the first tab having a releasable connector (6,406,506,606) at a distal end;
said foot wrap also having a second tab (4,407,507,607) formed from at least one of said two sheets for releasably securing the device to the foot about the heel, the second tab being generally perpendicular to the first tab, the second tab having a releasable connector (8,408,608) at a distal end; and
said main portion having exterior surface portion(s) on at least one of said sheets compatible with said releasable connectors so that a connection may be formed when said connectors are pressed against a said surface portion(s);
pressure sensing means (40A,40B) for sensing an amount of pressure supplied by said source; and
control means in communication with said pressure sensing means to adjust the amount of pressure supplied by said source.

2. A medical apparatus as claimed in claim 1, wherein the foot wrap is adapted such that, in use, during preparation for application of the wrap to the foot and the main portion are placed adjacent one another so that the second tab is substantially parallel to an axis of the foot but offset from the heel.

3. A medical apparatus as claimed in either of claims 1 or 2, wherein the two sheets are formed from dissimilar materials to one another.

4. A medical apparatus as claimed in claim 1, further comprising:
a housing (20) for releasably mounting said source on a foot board (900) of a standard hospital bed, said housing comprising a pair of opposite, retractable mounting hooks (21A, 21B) having sloped surfaces for biasing the source toward said foot board and having knobs (241) at distal ends thereof for helping retain the hooks on the foot board;
said hooks further having a horizontal bearing surface for engaging a top of foot boards having smaller widths.

5. A medical apparatus as claimed in either of claims 2 or 3, wherein the apparatus is adapted for affecting blood circulation, the apparatus comprising:
the wrap (1A,1B,1A',401,501,601) having the inflatable bladder (9A,9B,9,409,509,609) for applying pressure to a patient's extremity when said bladder is inflated;
a pressure source (501) for supplying pressurized fluid to inflate said bladder; and
a control (4) for controlling the inflation of said bladder in a cyclic manner wherein a plurality of successive cycles each include an inflation period followed by a deflation period, said control being adapted for gradually increasing the amount of pressure applied by said wrap over a series of the successive cycles.

6. A medical apparatus as claimed in claim 5, wherein said wrap comprises a foot wrap for applying pressure to a patient's foot (100).

7. A medical apparatus as claimed in claim 5, wherein:
said control includes a valve (36A,36B) in fluid communication between said pressure source and said bladder such that opening the valve for an open period causes inflation of the bladder, whereby the amount of pressure applied during the inflation period of a given cycle is related to a corresponding open period of the valve; and
said control is further adapted to gradually increase the applied pressure amounts of the series of successive cycles by gradually increasing the open period corresponding to each successive cycle in the series; the gradual increase in open period being from an initial open period corresponding to the first cycle of the series to a subsequent open period corresponding to the last cycle of the series; the subsequent open period being more than the initial open period.

8. A medical apparatus as claimed in claim 5, wherein said control is further adapted to prevent application of pressure in an amount exceeding a target pressure level.

9. A medical apparatus as claimed in claim 5, further comprising a pressure sensor (40A,40B) for sensing the pressure supplied of the pressurized fluid supplied by said pressure source to inflate the bladder; and
wherein said control is in communication with said pressure sensor and is further adapted to prevent application of pressure in an amount exceeding a target pressure level.

10. A medical apparatus as claimed in claim 9, wherein the control is further adapted to enable adjustment of the target pressure level.

11. A medical apparatus as claimed in claim 5, further comprising: a pressure sensor (40A,40B) for sensing the pressure of the pressurized fluid supplied by said pressure source to inflate the bladder; and
wherein said control is in communication with said pressure sensor and is further adapted for adjusting the amount of pressure to which the bladder is inflated by said pressure source based on pressures sensed by said pressure sensor.

12. A medical apparatus as claimed in claim 11, wherein said control is adapted for adjusting the amount of pressure to which the bladder is inflated by said pressure source based on the amount of pressure being sensed by said pressure sensor during the then current cycle.

13. A medical apparatus as claimed in claim 12, wherein:
said pressure sensor is adapted for taking a measurement of pressure supplied by said pressure source and converting said measurement into a voltage signal; and further comprising:
spike elimination means for preventing said pressure sensor from measuring an initial surge of pressure before said pressure stabilizes.

14. A medical apparatus as claimed in claim 13, wherein said spike elimination means further comprises at least one restrictor (34A,34B) and expansion tank (33A,33B) to decrease the flow of air pressure and provide an additional path for expansion of said initial surge of pressure.

15. A medical apparatus as claimed in claim 13, wherein said control further comprises an analog to digital converter to convert said voltage signal into a digital signal.

16. A medical apparatus as claimed in claim 15, wherein said control further comprises:
a micro-controller (41); and
a plurality of solenoid valves (36A, 36B, 32A, 32B) controlled by said micro-controller to adjust the amount of pressure supplied by said pressure source to said foot wrap;
said micro-controller being operable to read said digital signal for determining when to open and close said solenoid valves.

17. A medical apparatus as claimed in claim 16, wherein said control further comprises an error detection means for determining when pressure supplied by said pressure source is either too high or to low.

18. A medical apparatus as claimed in claim 16, wherein said control further comprises:
an operator console (42) having a liquid crystal display (46) and programmable keys (44A,44B,44C) to enter user settings;
memory means for storing said user settings to be processed by said micro-controller.

19. A medical apparatus as claimed in claim 5, wherein said pressure source comprises:
an air compressor (38); and
an accumulator (37) for storing compressed air received from said air compressor.

20. A medical apparatus as claimed in any preceding claim, wherein, in use, the second tab connects to an exterior surface portion provided on the first tab.

21. A flexible fabric foot wrap (1A,1B,1A', 401,501,601) formed from two sheets (3,4) and having an inflatable bladder (9A,9B,409,509,609) therein;
a connector (11,411,511) opening into the inflatable bladder and suitable for connection to a source of pressurized fluid;
said foot wrap having a main portion including a first tab (5,405,505,605) formed from at least one of said two sheets the main portion being adapted for releasably securing the device to a human foot (100) about an arch thereof, the first tab having a releasable connector (6,406,506,606) at a distal end;
said foot wrap also having a second tab (4,407,507,607) formed from at least one of said two sheets for releasably securing the device to the foot about the heel, the second tab being generally perpendicular to the first tab, the second tab having a releasable connector (8,408,608) at a distal end; and
said main portion having exterior surface portion(s) on at least one of said sheets compatible with said releasable connectors so that a connection may be formed when said connectors are pressed against a said surface portion(s).

22. A flexible fabric foot wrap as claimed in claim 21, wherein the wrap is generally T-shaped.

## Patentansprüche

1. Medizinisches Gerät (500) zur zyklischen Ausübung von Druck auf einen Fuß (100), das folgendes aufweist:
eine Fußbandage (1A, 1B, 1A', 401, 501, 601) aus flexiblem Gewebe, die aus zwei Lagen (3, 4) gebildet wird und in der sich eine aufblasbare Blase (9A, 9B, 409, 509, 609) befindet,
einen Anschluß (11, 411, 511), der sich in die aufblasbare Blase öffnet und für die Verbindung mit einer Quelle für ein Druckfluid geeignet ist,
wobei die Fußbandage einen Hauptabschnitt hat, der eine erste Lasche (5, 405, 505, 605) einschließt, die aus wenigstens einer der beiden Lagen gebildet wird, wobei der Hauptabschnitt dafür ausgelegt ist, die Vorrichtung lösbar am Fuß (100) eines Menschen um einen Spann desselben zu befestigen, wobei die erste Lasche an einem distalen Ende ein lösbares Verbindungselement (6, 406, 506, 606) hat,
wobei die Fußbandage auch eine zweite Lasche (4, 407, 507, 607) hat, die aus wenigstens einer der beiden Lagen gebildet wird, um die Vorrichtung um die Ferse lösbar am Fuß zu befestigen, wobei die zweite Lasche allgemein senkrecht zur ersten Lasche ist, wobei die zweite Lasche ein lösbares Verbindungselement (8, 408, 608) an einern distalen Ende hat, und
wobei der Hauptabschnitt auf wenigstens einer der Lagen (einen) äußeren Oberflächenabschnitt(e) hat, der mit den lösbaren Verbindungselementen kompatibel ist, so daß eine Verbindung hergestellt werden kann, wenn die Verbindungselemente gegen den (die) Oberflächenabschnitt(e) gedrückt werden,
Druckmeßmittel (40A, 40B) zur Messung einer Größe des Drucks, der durch die Quelle zugeführt wird, und
Steuerungsmittel in Verbindung mit den Druckmeßmitteln, um die Größe des Drucks zu regulieren, der durch die Quelle zugeführt wird.

2. Medizinisches Gerät nach Anspruch 1, bei dem die Fußbandage angepaßt ist, derartig, daß bei der Vorbereitung für das Anlegen der Bandage am Fuß dieser und der Hauptabschnitt nebeneinander angeordnet werden, so daß die zweite Lasche im wesentlichen parallel zu einer Achse des Fußes, aber gegenüber der Ferse versetzt, ist.

3. Medizinisches Gerät nach einem der Ansprüche 1 oder 2, bei dem die beiden Lagen aus unterschiedlichen Materialien hergestellt werden.

4. Medizinisches Gerät nach Anspruch 1, das außerdem folgendes aufweist:
ein Gehäuse (20) zur lösbaren Anbringung der Quelle an einem Fußteil (900) eines Standard-Krankenhausbettes, wobei das Gehäuse ein Paar gegenüberliegender, einziehbarer Befestigungshaken (21A, 21B) aufweist, die geneigte Flächen haben, um die Quelle zum Fußteil hin einseitig einzuspannen, und die an deren distalen Enden Knöpfe (241) haben, um das Halten der Haken am Fußteil zu unterstützen,
wobei die Haken außerdem eine waagerechte Auflagefläche zum Ineinandergreifen mit einem Oberteil eines Fußteils mit einer geringeren Breite haben.

5. Medizinisches Gerät nach einem der Ansprüche 2 oder 3, bei dem das Gerät dafür ausgelegt ist, die Blutzirkulation zu beeinflussen, wobei das Gerät folgendes aufweist:
die Bandage (1A, 1B, 1A', 401, 501, 601) mit der aufblasbaren Blase (9A, 9B, 9, 409, 509, 609) zum Ausüben von Druck auf eine Extremität eines Patienten, wenn die Blase aufgeblasen wird,
eine Druckquelle (501) zur Zuführung von Druckfluid zum Aufblasen der Blase und
eine Steuerung (4) zur Steuerung des Aufblasens der Blase auf zyklische Weise, worin eine Vielzahl von aufeinanderfolgenden Zyklen jeweils eine Aufblasperiode, gefolgt von einer Abblasperiode, einschließt, wobei die Steuerung dafür ausgelegt ist, die Größe des Drucks, welcher der Bandage über eine Reihe von aufeinanderfolgenden Zyklen zugeführt wird, allmählich zu vergrößern.

6. Medizinisches Gerät nach Anspruch 5, bei dem die Bandage eine Fußbandage zur Ausübung von Druck auf einen Fuß (100) eines Patienten umfaßt.

7. Medizinisches Gerät nach Anspruch 5, bei dem:
die Steuerung ein Ventil (36A, 36B) in Fluidverbindung zwischen der Druckquelle und der Blase einschließt, derartig, daß das Öffnen des Ventils für eine Öffnungsperiode das Aufblasen der Blase bewirkt, wodurch die Größe des Drucks, der während der Aufblasperiode eines gegebenen Zyklusses ausgeübt wird, zu einer entsprechenden Öffnungsperiode des Ventils in Beziehung gesetzt wird, und
wobei die Steuerung außerdem dafür ausgelegt ist, die Größe des ausgeübten Drucks in der Reihe von aufeinanderfolgenden Zyklen dadurch allmählich zu vergrößern, daß allmählich die Öffnungsperiode, die jedem aufeinanderfolgenden Zyklus in der Reihe entspricht, verlängert wird, wobei die allmähliche Verlängerung in der Öffnungsperiode von einer anfänglichen Öffnungsperiode, die dem ersten Zyklus der Reihe entspricht, bis zu einer nachfolgenden Öffnungsperiode, die dem letzten Zyklus der Reihe entspricht, erfolgt, wobei die nachfolgende Öffnungsperiode länger als die anfängliche Öffnungsperiode ist.

8. Medizinisches Gerät nach Anspruch 5, bei dem die Steuerung außerdem dafür ausgelegt ist, die Anwendung von Druck in einer Größe, die einen Zieldruckpegel übersteigt, zu verhindern.

9. Medizinisches Gerät nach Anspruch 5, das außerdem einen Drucksensor (40A, 40B) zum Messen des zugeführten Drucks des Druckfluids aufweist, das von der Druckquelle zugeführt wird, um die Blase aufzublasen, und
bei dem die Steuerung mit dem Drucksensor in Verbindung steht und außerdem dafür ausgelegt ist, die Anwendung von Druck in einer Größe, die einen Zieldruckpegel übersteigt, zu verhindern.

10. Medizinisches Gerät nach Anspruch 9, bei dem die Steuerung außerdem dafür ausgelegt ist, die Regulierung des Zieldruckpegels zu ermöglichen.

11. Medizinisches Gerät nach Anspruch 5, das außerdem einen Drucksensor (40A, 40B) zum Messen des Drucks des Druckfluids aufweist, das von der Druckquelle zugeführt wird, um die Blase aufzublasen, und
bei dem die Steuerung mit dem Drucksensor in Verbindung steht und außerdem dafür ausgelegt ist, die Größe des Drucks zu regulieren, auf den die Blase durch die Druckquelle auf der Grundlage der Drücke, die durch den Drucksensor gemessen werden, aufgeblasen wird.

12. Medizinisches Gerät nach Anspruch 11, bei dem die Steuerung dafür ausgelegt ist, die Größe des Drucks zu regulieren, auf den die Blase durch die Druckquelle auf der Grundlage der Größe des Drucks, der durch den Drucksensor während des gegenwärtigen Zyklusses gemessen wird, aufgeblasen wird.

13. Medizinisches Gerät nach Anspruch 12, bei dem:
der Drucksensor dafür ausgelegt ist, eine Messung des durch die Druckquelle zugeführten Drucks vorzunehmen und diese Messung in ein Spannungssignal umzuwandeln, und das außerdem folgendes aufweist:
Druckspitzen-Ausschaltungsmittel, um die Messung eines anfänglichen Druckstoßes durch den Drucksensor zu verhindern, bevor sich der Druck stabilisiert.

14. Medizinisches Gerät nach Anspruch 13, bei dem die Druckspitzen-Ausschaltungsmittel außerdem wenigstens eine Drossel (34A, 34B) und ein Ausdehnungsgefäß (33A, 33B) aufweisen, um den Strom des Luftdrucks zu verringern und eine zusätzliche Ausdehnungsbahn für den anfänglichen Druckstoß bereitzustellen.

15. Medizinisches Gerät nach Anspruch 13, bei dem die Steuerung außerdem einen Analog-Digital-Wandler aufweist, um das Spannungssignal in ein Digitalsignal umzuwandeln.

16. Medizinisches Gerät nach Anspruch 15, bei dem die Steuerung außerdem folgendes aufweist:
einen Mikroregler (41) und
eine Vielzahl von Magnetventilen (36A, 36B, 32A, 32B), die durch den Mikroregler gesteuert werden, um die Größe des Drucks zu regulieren, der durch die Druckquelle der Fußbandage zugeführt wird,
wobei der Mikroregler dahingehend funktionsfähig ist, das Digitalsignal zu lesen, um zu bestimmen, wann die Magnetventile zu öffnen und zu schließen sind.

17. Medizinisches Gerät nach Anspruch 16, bei dem die Steuerung außerdem ein Fehler-Erkennungsmittel aufweist, um zu bestimmen, wenn der durch die Druckquelle zugeführte Druck entweder zu hoch oder zu niedrig ist.

18. Medizinisches Gerät nach Anspruch 16, bei dem die Steuerung außerdem folgendes aufweist:
eine Bedienungstafel (42) mit einer Flüssigkristall-Anzeige (46) und programmierbaren Tasten (44A, 44B, 44C) zur Eingabe von Anwendereinstellungen,
Speichermittel zur Speicherung der Anwendereinstellungen, die durch den Mikroregler zu verarbeiten sind.

19. Medizinisches Gerät nach Anspruch 5, bei dem die Druckquelle folgendes aufweist:
einen Drucklufterzeuger (38) und
einen Druckluftspeicher (37) zur Speicherung von Druckluft, die von dem Drucklufterzeuger empfangen worden ist.

20. Medizinisches Gerät nach einem der vorhergehenden Ansprüche, bei dem bei der Anwendung die zweite Lasche mit einem äußeren Oberflächenabschnitt verbunden wird, der auf der ersten Lasche bereitgestellt wird.

21. Fußbandage (1A, 1B, 1A', 401, 501, 601) aus flexiblem Gewebe, die aus zwei Lagen (3, 4) gebildet wird und in der sich eine aufblasbare Blase (9A, 9B, 409, 509, 609) befindet,
einen Anschluß (11, 411, 511), der sich in die aufblasbare Blase öffnet und für die Verbindung mit einer Quelle für ein Druckfluid geeignet ist,
wobei die Fußbandage einen Hauptabschnitt hat, der eine erste Lasche (5, 405, 505, 605) einschließt, die aus wenigstens einer der beiden Lagen gebildet wird, wobei der Hauptabschnitt dafür ausgelegt ist, die Vorrichtung lösbar am Fuß (100) eines Menschen um einen Spann desselben zu befestigen, wobei die erste Lasche an einem distalen Ende ein lösbares Verbindungselement (6, 406, 506, 606) hat,
wobei die Fußbandage auch eine zweite Lasche (4, 407, 507, 607) hat, die aus wenigstens einer der beiden Lagen gebildet wird, um die Vorrichtung lösbar um die Ferse am Fuß zu befestigen, wobei die zweite Lasche allgemein senkrecht zur ersten Lasche ist, wobei die zweite Lasche ein lösbares Verbindungselement (8, 408, 608) an einem distalen Ende hat, und
wobei der Hauptabschnitt auf wenigstens einer der Lagen (einen) äußere(n) Oberflächenabschnitt(e) hat, der mit den lösbaren Verbindungselementen kompatibel ist, so daß eine Verbindung hergestellt werden kann, wenn die Verbindungselemente gegen den (die) Oberflächenabschnitt(e) gedrückt werden.

22. Fußbandage aus flexiblem Gewebe nach Anspruch 21, bei dem die Bandage allgemein T-förmig ist.

## Revendications

1. Dispositif médical (500) destiné à l'application cyclique d'une pression à un pied (100), comprenant:
un bandage de pied en tissu flexible (1A, 1B, 1A', 401, 501, 601) formé à partir de deux feuilles (3,4) et contenant une vessie gonflable (9A, 9B, 409, 509, 609);
un raccord (11, 411, 511) ouvert vers la vessie gonflable et destiné à être relié à une source de fluide sous pression;
ledit bandage du pied comportant une partie principale englobant une première languette (5, 405, 505, 605) formée à partir d'au moins une desdites deux feuilles, la partie principale étant destinée à fixer de manière amovible le dispositif à un pied humain (100) autour de la voûte plantaire, la première languette comportant un raccord amovible (6, 406, 506, 606) au niveau d'une extrémité distale;
ledit bandage du pied comportant également une deuxième languette (4, 407, 507, 607) formée à partir d'au moins une desdites deux feuilles, pour fixer de manière amovible le dispositif au pied autour du talon, la deuxième languette étant en général perpendiculaire à la première languette, la deuxième languette comportant un raccord amovible (8, 408, 608) au niveau d'une extrémité distale; et
ladite partie principale comportant une (des) partie(s) de surface externe(s) sur au moins une desdites feuilles, compatible(s) avec lesdits raccords amovibles, de sorte qu'une liaison peut être formée lorsque lesdits raccords sont pressés contre une (des) dite(s) partie(s) de surface;
un moyen de détection de la pression (40A, 40B) pour détecter une quantité de pression fournie par ladite source; et
un moyen de commande communiquant avec ledit moyen de détection de la pressiion pour ajuster la quantité de pression fournie par ladite source.

2. Dispositif médical selon la revendication 1, dans lequel le bandage du pied est conçu de sorte qu'en service, au cours de la préparation de l'application du bandage sur le pied, le bandage et la partie principale sont placés en des points adjacents l'un à l'autre, de sorte que la deuxième languette est pratiquement parallèle à un axe du pied, mais décalée par rapport au talon.

3. Dispositif médical selon l'une des revendications 1 ou 2, dans lequel les deux feuilles sont formées à partir de matériaux différents.

4. Dispositif médical selon la revendication 1, comprenant en outre:
un boîtier (20) pour le montage amovible de ladite source sur un repose-pied (90) d'un lit d'hôpital standard, ledit boîtier comprenant une paire de crochets de montage rétractables opposés (21A, 21B) comportant des surfaces inclinées pour pousser la source vers ledit repose-pied et comportant des boutons (241) au niveau des extrémités distales correspondantes pour faciliter la retenue des crochets sur le repose-pied;
lesdits crochets comportant en outre une surface de support horizontale en vue de l'engagement dans une partie supérieure de repose-pied ayant des largeurs réduites.

5. Dispositif médical selon l'une des revendications 2 ou 3, dans lequel le dispositif est destiné à influencer la circulation sanguine, le dispositif comprenant:
le bandage (1A, 1B, 1A', 401, 501, 601) comportant la vessie gonflable (9A, 9B, 9, 409, 509, 609) pour appliquer une pression à une extrémité d'un patient lorsque ladite vessie est gonflée;
une source de pression (501) pour fournir du fluide sous pression en vue du gonflement de ladite vessie; et
une unité de commande (4) pour assurer la commande du gonflement de ladite vessie de manière cyclique, plusieurs cycles successifs englobant chacun une période de gonflement suivie par une période de dégonflement, ladite unité de commande étant destinée à accroître progressivement la quantité de pression appliquée par ledit bandage pendant une série de cycles successifs.

6. Dispositif médical selon la revendication 5, dans lequel ledit bandage comprend un bandage de pied destiné à appliquer une pression au pied d'un patient (100).

7. Dispositif médical selon la revendication 5, dans lequel:
ladite unité de commande englobe une vanne (36A, 36B) établissant une communication de fluide entre ladite source de pression et ladite vessie, de sorte que l'ouverture de la vanne pendant une période ouverte entraîne le gonflement de la vessie, la quantité de pression appliquée au cours de la période de gonflement d'un cycle défini dépendant ainsi d'une période d'ouverture correspondante de la vanne; et
ladite unité de commande étant en outre destinée à accroître progressivement les quantités de pression appliquées des séries de cycles successifs par accroissement progressif de la période d'ouverture correspondant à chaque cycle successif dans la série; l'accroissement progressif de la période d'ouverture allant d'une période d'ouverture initiale, correspondant au premier cycle de la série, à une période d'ouverture ultérieure correspondant au dernier cycle de la série; la période d'ouverture ultérieure étant supérieure à la période d'ouverture initiale.

8. Dispositif médical selon la revendication 5, dans lequel ladite unité de commande est destinée en outre à empêcher l'application d'une quantité de pression supérieure à un niveau de pression cible.

9. Dispositif médical selon la revendication 5, comprenant en outre un détecteur de pression (40A, 40B) pour détecter la pression du fluide sous pression fourni par ladite source de pression pour gonfler la vessie; et
ladite unité de commande étant en communication avec ledit détecteur de pression et étant en outre destinée à empêcher l'application d'une quantité de pression supérieure à un niveau de pression cible.

10. Dispositif médical selon la revendication 9, dans lequel l'unité de commande est destinée en outre à permettre l'ajustement du niveau de pression cible.

11. Dispositif médical selon la revendication 5, comprenant en outre: un détecteur de pression (40A, 40B) pour détecter la pression du fluide sous pression fourni par ladite source de pression pour gonfler la vessie; et
ladite unité de commande étant en communication avec ledit détecteur de pression et étant destinée en outre à ajuster la quantité de pression de gonflement de la vessie par ladite source de pression sur la base des pressions détectées par ledit détecteur de pression.

12. Dispositif médical selon la revendication 11, dans lequel ladite unité de commande est destinée à ajuster la quantité de pression de gonflement de la vessie par ladite source de pression sur la base de la quantité de pression détectée par ledit détecteur de pression au cours du cycle de service.

13. Dispositif médical selon la revendication 12, dans lequel:
ledit détecteur de pression est destiné à prendre une meure de la pression fournie par ladite source de pression et à convertir ladite mesure en un signal de tension; et comprenant en outre:
un moyen d'élimination de pointes, pour empêcher que ledit détecteur de pression ne mesure une saute initiale de la pression avant la stabilisation de ladite pression.

14. Dispositif médical selon la revendication 13, dans lequel ledit moyen d'élimination des pointes comprend en outre au moins un restricteur (34A, 34B) et un réservoir de détente (33A, 33B) pour réduire l'écoulement de la pression d'air et pour établir une trajectoire additionnelle pour la détente de ladite saute initiale de la pression.

15. Dispositif médical selon la revendication 13, dans lequel ladite unité de commande comprend en outre un convertisseur analogique-numérique pour convertir ledit signal de tension en un signal numérique.

16. Dispositif médical selon la revendication 15, dans lequel ladite unité de commande comprend en outre:
un micro-contrôleur (41); et
plusieurs vannes électromagnétiques (36A, 36B, 32A, 32B) commandées par ledit micro-contrôleur pour ajuster la quantité de pression fournie par ladite source de pression audit bandage de pied;
ledit micro-contrôleur étant destiné à lire ledit signal numérique pour déterminer le moment d'ouverture et de fermeture desdites vannes électromagnétiques.

17. Dispositif médical selon la revendication 16, dans lequel ladite unité de commande comprend en outre un moyen de détection des erreurs destiné à déterminer si la pression fournie par ladite source de pression est trop élevée ou trop basse.

18. Dispositif médical selon la revendication 16, dans lequel ladite unité de commande comprend en outre:
une console d'opérateur (42) comportant un affichage à cristaux liquides (46) et des touches programmables (44A, 44B, 44C) pour entrer les réglages de l'utilisateur;
un moyen de mémoire pour enregistrer lesdits réglages de l'utilisateur en vue d'un traitement par ledit micro-contrôleur.

19. Dispositif médical selon la revendication 5, dans lequel ladite source de pression comprend:
un compresseur d'air (38); et
un accumulateur (37) pour stocker l'air comprimé fourni par ledit compresseur d'air.

20. Dispositif médical selon l'une quelconque des revendications précédentes, dans lequel la deuxième languette est reliée en service à une partie de surface externe agencée sur la première languette.

21. Bandage de pied en tissu flexible (1A, 1B, 1A', 401, 501, 601) formé à partir de deux feuilles (3, 4) et contenant une vessie gonflable (9A, 9B, 409, 509, 609);
un raccord (11, 411, 511) ouvert vers ladite vessie gonflable et destiné à être relié à une source de fluide sous pression;
ledit bandage de pied comportant une partie principale englobant une première languette (5, 405, 505, 605) formée à partir d'au moins une desdites deux feuilles, la partie principale étant destinée à fixer de manière amovible le dispositif à un pied humain (100), autour de la voûte plantaire, la première languette comportant un raccord amovible (6, 406, 506, 606) au niveau d'une extrémité distale;
ledit bandage de pied comportant également une deuxième languette (4, 407, 507, 607) formée à partir d'au moins une desdites deux feuilles pour fixer de manière amovible le dispositif sur le pied, autour du talon, la deuxième languette étant en général perpendiculaire à la première languette, la deuxième languette comportant un raccord amovible (8, 408, 608) au niveau d'une extrémité distale; et
ladite partie principale comportant une (des) partie(s) de surface externe (s) sur au moins une desdites feuilles, compatible(s) avec lesdits raccords amovibles, de sorte qu'une liaison peut être établie lorsque lesdits raccords sont pressés contre une (des) dite(s) partie(s) de surface.

22. Bandage de pied en tissu flexible selon la revendication 21, le bandage ayant en général une forme en T.
